# EUROPEAN PATENT APPLICATION

(11) **EP 3 315 495 A1**
(43) Date of publication of application: **02.05.2018**
(21) Application number: 16195622.2
(22) Date of filing: 25.10.2016
(51) Int. Cl.: C07D 263/56, C07D 235/12, C07D 235/14, C07D 277/64, A61K 31/4184, A61K 31/423, A61K 31/427, A61P 29/00

(54) **N,N' -DIARYLUREA, N,N' -DIARYLTHIOUREA AND N,N' -DIARYLGUANIDINO COMPOUNDS FOR USE IN TREATMENT AND PREVENTION OF INFLAMMATORY DISEASE**

(71) Applicant: Universität Innsbruck, 6020 Innsbruck (AT); Friedrich-Schiller-Universität Jena, 07737 Jena (DE); Institute of Experimental Botany, Academy of Sciences of the Czech Republic, 165 02 Prague 6 - Lysolaje (CZ)
(72) Inventor: Schuster, Daniela, 6410 Telfs (AT); Temml, Veronika, 6020 Innsbruck (AT); Kutil, Zsofia, 16000 Praha 6 (CZ); Matuszczak, Barbara, 6020 Innsbruck (AT); Werz, Oliver, 07749 Jena (DE); Garscha, Ulrike, 99423 Weimar (DE); Waltenberger, Birgit, 6020 Innsbruck (AT); Stuppner, Hermann, 6091 Götzens (AT)
(74) Representative: Schwarz & Partner Patentanwälte OG

(57) **Abstract**

A compound according to formula I 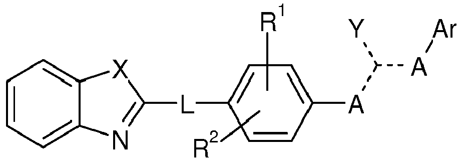 wherein L is CH₂-O, CH₂-S, CH₂-CH₂, or CH=CH; R¹ and R² are independently selected out of H, halogen, C1 to C3 alkyl, halogenated C1 to C3 alkyl, C1 to C3 alkyloxy, or halogenated C1 to C3 alkyloxy; Ar is an aryl moiety, preferably phenyl or naphthyl, or a moncyclic- or bicyclic heteroaryl moiety, wherein optionally the aryl or heteroaryl moiety is further substituted; X is O, S, or NR³, wherein R³ is H, C1 to C3 alkyl, halogenated C1 to C3 alkyl, or *tert*-butyloxycarbonyl; Y is O, S, NR⁴ or N(R⁴)R⁵, wherein R⁴ and R⁵ are independently selected out of H or C1 to C3 alkyl; and wherein if Y is O, S, or NR⁴, then both A are NH, and the punctuated bonds represent a double bond to Y and single bonds to each A; or wherein if Y is N(R⁴)R⁵, then one A is N, the other A is NH, and the punctuated bonds represent a double bond to the A being N, a single bond to the A being NH and a single bond to Y; and use of such a compound in treatment or prevention of an inflammatory disease or an inflammation-related disorder.

## Description

The invention relates to diarylurea, diarylthiourea and diarylguanidino compounds as well as their use in treatment and prevention of an inflammatory disease or an inflammation-related disorder. Moreover, the invention relates to a process for synthesis of diarylurea and diarylthiourea compounds.

### BACKGROUND OF THE INVENTION

Inflammation is a process triggered by connective tissue to react to external (mechanic, physical, chemical) and internal (microorganism, metabolites, tumors) stimuli. It is aimed at terminating the stimulus and repairing tissue damage. The classic symptoms of inflammation are skin reddening, localized rise in temperature, pain, swelling, and loss of function. While inflammation is in general a useful process to eliminate specific noxae, misdirected inflammatory processes lead to a variety of often chronic pathologies such as rheumatoid arthritis, asthma, atherosclerosis, and even neurodegenerative diseases such as Alzheimer's and Parkinson's disease.

The arachidonic acid (AA) cascade is an important pathway in pharmaceutical research to counter inflammation. AA is the precursor for various inflammation-regulating lipid mediators and multiple enzymes are involved in the synthesis and degradation of the effector molecules.

So far, cyclooxygenase 1 and 2 (COX1, COX2) are the best explored points of attack within the AA cascade. COX inhibitors are available and dominate the market of anti-inflammatory drugs (e.g. ibuprofen, acetylsalicylic acid, naproxen). There is also an approved inhibitor for 5-lipoxygenase (zileuton) and antagonists for the cysteinyl leukotriene recpetor (e.g., montelukast). Additionally, there are multiple compounds targeting other enzymes within the AA cascade that are currently undergoing clinical trials.

One of these enzymes is soluble epoxide hydrolase (sEH) which catalyses degradation of epoxy-eicosatrienoic acids (EETs). The EETs are formed by members of the CYP450 superfamily, i.e. CYP2C and CYP2J and are known to have strong anti-inflammatory effects.

In contrast, the degradation products, dihydroxyeicosatrienoic acids (DHETs), do not display the same anti-inflammatory effects. EETs have also been shown to be analgesic and antihypertensive, and to play a critical role in cardiovascular physiology. Thus, sEH is considered as an interesting target for various pathologies (Shen, H.C.; Expert Opin Ther Pat. 2010 (7) 941-956. doi: 10.1517/13543776.2010.484804).

Typical scaffolds of sEH inhibitors include urea moieties, especially *N,N'*-dialkyl urea, like e.g. *N,N'*-dicyclohexylurea (DCU), *N*-adamantyl-*N'*-dodecanoic acid urea (AUDA), *trans*-4-[4-(*N*-adamantan-*N'*-yl-ureido)-cyclohexyloxy]-benzoic acid]) t-AUCB, or *N*-alkyl-*N*'-aryl urea compounds, e.g. *N*-trifluoromethoxyphenyl-3-(*N*'-acetylpiperidin-4-yl) urea TPAU.

Another promising target in the AA cascade is 5-lipoxygenase (5-LOX) as well as its 5-LOX-activating protein (FLAP). FLAP is a membrane-associated protein, which is essential for 5-LOX activity in the cellular environment as it facilitates the transfer of AA, released by cytosolic phospholipase (cPLA₂), to the activated and nuclear membrane bound 5-LOX. A reduced 5-LOX activity (either by direct inhibition or indirectly via FLAP inhibition) results in decreased pro-inflammatory leukotriene (LT) synthesis. Consequently, inhibition of the 5-LOX pathway allows to reduce inflammation signs. However, only the direct 5-LOX inhibitor zileuton is approved as drug against asthma in US, but with severe side effects. Severe side effects due to unspecific interaction of direct 5-LOX inhibitors with other redox-active biological systems are the reason for the poor availability of candidate compounds for clinical developments.

In this context, it should be noted that experimental distinction between 5-LOX inhibition and FLAP inhibition is not trivial as FLAP inhibition may result in similar experimental read out as 5-LOX inhibition and may only be tested indirectly (Pergola, C.; Gerstmeier, J.; Monch, B.; Caliskan, B.; Luderer, S.; Weinigel, C.; Barz, D.; Maczewsky, J.; Pace, S.; Rossi, A.; Sautebin, L.; Banoglu, E.; Werz, O., British Journal of Pharmacology 2014, 171 (12), 3051-3064. doi 10.1111/bph.126525).

Molecules inhibiting 5-LOX activity by interference with FLAP functionality are summarized for example by Pettersen et al (Pettersen, D.; Davidsson, Ö.; Whatling, C.; Bioorg Med Chem Lett. 2015, 25 (13), 2607-2612. doi: 10.1016/j.bmcl.2015.04.090). First generation clinical FLAP inhibitors share an acidic function as common feature. Recent FLAP inhibitor generations are characterized by improved pharmacokinetics and often show a diaryl-sub structure.

Recently, in several cases it has been found warranted to inhibit the inflammatory process in multiple nodes. Addressing multiple targets may allow to work towards low dose treatment that causes fewer side effects. (Hwang S.H., Wecksler A.T. Wagner K, Hammock BD, Curr Med Chem. 2013, 20 (13), 1783-1799, doi: 10.2174/0929867311320130013). Various dual inhibitors of COX and 5-LOX were investigated. Licofelone, a compound promoted as COX/LOX inhibitor turned out to actually target FLAP and COX.

Furthermore COX2/sEH dual inhibitors were described, which show a pyrazole substructure from celecoxib and an urea motive known from sEH inhibitors (Hwang S.H., Wagner K, Morriseau C., Liu, J.Y., DON H., Wecksler A.T., Hammock BD, J Med Chem. 2011, 54 (8), 3037-3050. doi: 10.1021/jm2001376.).

Identification of dual inhibitors of 5-LOX and sEH by *in silico* modeling has been described (Moser D., Wisniewska J.M., Hahn S., Achenbach J., La Buscaó E., Klingler F.M., Hofmann, B., Steinhilber D., Proschak E. ACS Med Chem Lett. 2012; 3 (2), 155-158. doi: 10.1021/ml200286e). A dual inhibition of the 5-LOX pathway and sEH pathway is considered desirable. Also the combined administration of FLAP inhibitor MK886 and the sEH inhibitor AUDA-BE was described in US 2010-0286222.

However, no dual inhibitors of FLAP and sEH are available so far. Moreover, alternative scaffolds for FLAP or sEH inhibitors that may overcome the pharmacodynamic issues are considered attractive.

### DESCRIPTION OF THE INVENTION

The present invention relates to compounds according to the generic formula I 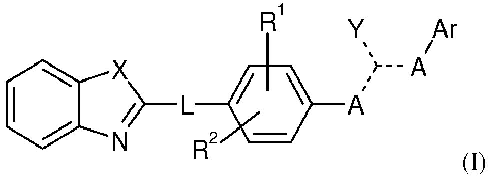
wherein L is CH₂-O, CH₂-S, CH₂-CH₂, or CH=CH;
R¹ and R² are independently selected out of H (hydrogen), halogen, C1 to C3 alkyl, halogenated C1 to C3 alkyl, C1 to C3 alkyloxy, or halogenated C1 to C3 alkyloxy;
Ar is an aryl moiety, preferably phenyl or naphthyl, or a moncyclic or bicyclic heteroaryl moiety, wherein optionally the aryl or heteroaryl moiety is further substituted;
X is O, S, or NR³, wherein R³ is H, C1 to C3 alkyl, halogenated C1 to C3 alkyl, or *tert*-butyloxycarbonyl;
Y is O, S, NR⁴ or N(R⁴)R⁵, wherein R⁴ and R⁵ are independently selected out of H or C1 to C3 alkyl; and
wherein if Y is O, S, or NR⁴, then both A are NH, and the punctuated bonds represent a double bond to Y and single bonds to each A; or
wherein if Y is N(R⁴)R⁵, then one A is N, the other A is NH, and the punctuated bonds represent a double bond to the A being N, a single bond to the A being NH and a single bond to Y.

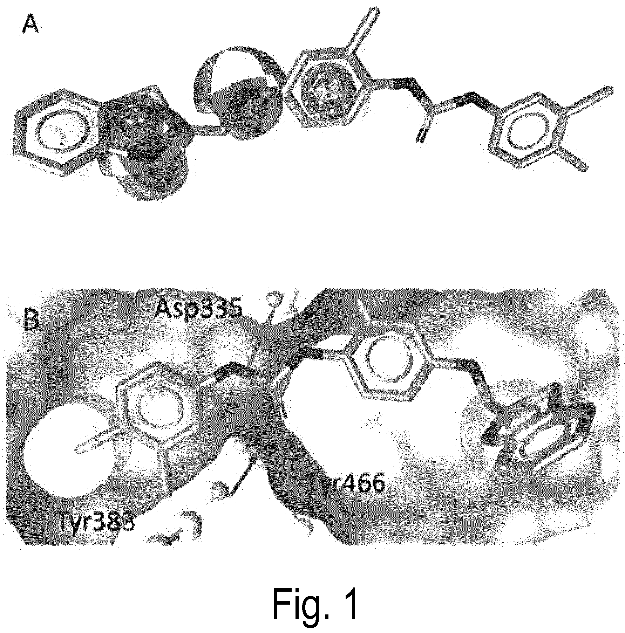

Formula I describes *N,N'*-diaryl substituted urea, thiourea or guanidino compounds. Initially, the inventors identified *N*-[4-(2-benzothiazolylmethoxy)-2-methylphenyl]-*N'*-(3,4-dichlorophenyl)-urea (Code: I-12, CAS: 724453-98-9, alternative nomenclature 1-[4-(2-benzothiazolylmethoxy)-2-methylphenyl]-3-(3,4-dichlorophenyl)-urea) as hit compound by a virtual screening campaign using 3D-pharmacophore models for FLAP and sEH (Example 1, Figure 1):

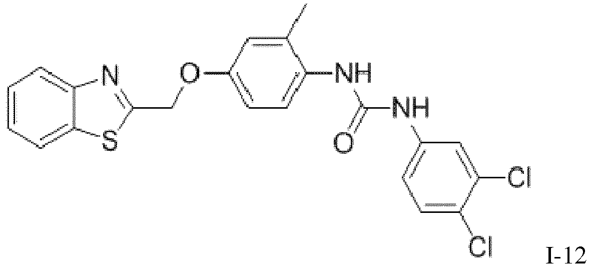

Biologically activity of this compound in terms of reduction of biosynthesis of leukotrienes (LTs) in cellular systems was confirmed (Example 2). The reduced level of the 5-LOX products in presence of I-12 was not related to direct 5-LOX inhibition but mediated by interference with FLAP as confirmed by several experiments (Example 5). Additionally, it was observed that this compound also inhibits the activity of sEH in a biochemical assay (Example 3). In an *in vivo* LT-mediated peritonitis model in mice the compound I-12 shows results similar to an established FLAP inhibitor and better than the 5-LOX inhibitor zileuton.

Subsequently, the inventors studied other compounds similar to I-12, wherein other compounds also showed promising *in vitro* activity regarding both sEH and FLAP (Table 1).

The inventors found that an amide from the [N-4-(2-benzothiazolylmethoxy)-2-methylphenyl]-amine may show FLAP inhibitory activity (I12-11). However, the compound with amide instead of urea or thiourea function did not show sEH inhibition. On the other side, a respective *N*-[4-(2-benzothiazolylmethoxy)-2-methylphenyl]-*N'*-cyclohexyl-urea (I12-2) compound did not show an effect on FLAP activity or 5-LOX activity. Without wishing to be bound by theory, the inventors attribute the special dual effect to the distinct scaffold of the generic formula I. The identification and provision of compounds that inhibit both FLAP function and sEH activity is highly desirable as additives or synergistic effects on the inflammatory signaling of the AA cascade and clinical symptoms of inflammation are expected.

Thus, in one aspect the invention relates to a compound according to formula I 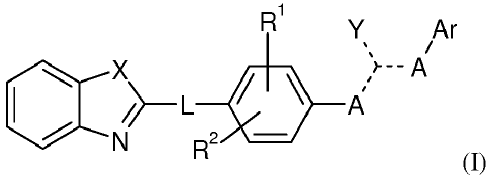
wherein L is CH₂-O, CH₂-S, CH₂-CH₂, or CH=CH;
R¹ and R² are independently selected out of H, halogen, C1 to C3 alkyl, halogenated C1 to C3 alkyl, C1 to C3 alkyloxy, or halogenated C1 to C3 alkyloxy;
Ar is an aryl moiety, preferably phenyl or naphthyl, or a moncyclic- or bicyclic heteroaryl moiety, wherein optionally the aryl or heteroaryl moiety is further substituted;
X is O, S, or NR³, wherein R³ is H, C1 to C3 alkyl, halogenated C1 to C3 alkyl, or *tert*-butyloxycarbonyl;
Y is O, S, NR⁴ or N(R⁴)R⁵, wherein R⁴ and R⁵ are independently selected out of H or C1 to C3 alkyl; and
wherein if Y is O, S, or NR⁴, then both A are NH, and the punctuated bonds represent a double bond to Y and single bonds to each A; or
wherein if Y is N(R⁴)R⁵, then one A is N, the other A is NH, and the punctuated bonds represent a double bond to the A being N, a single bond to the A being NH and a single bond to Y;
with the provision that a compound in which L is CH₂-O, X is NH, Y is S, both A are NH and both R¹ and R² are H is excluded,
for use as a medicament.

The provision excludes compounds according to formula I^{X}

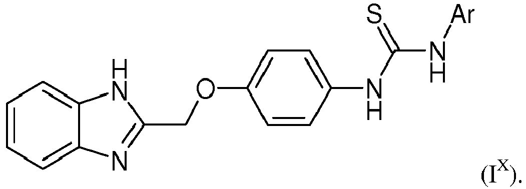

Individual compounds of formula I^{X} have been described before (Synthesis and neuropharmacological studies of some newer substituted benzimidazolyl thiocarbamides, Shukla, J.S.; Saxena, S.; Misra, R.; Indian J. Physiol. Pharmacol.; 1982, 26, 2, 176-178). The N'-benzimidazole oxymethylene phenyl-*N"*-phenyl thiocarbamides were studied as potential inhibitors of monoaminooxidase and in association with convulsions. In those compounds, when written according to general formula I, L is CH₂-O, X is NH, Y is S, both A are NH and both R¹ and R² are H. None of the thiourea compounds described in this reference was further substituted at the central 4-amino phenoxy moiety, i.e. R¹ and R² are hydrogen in all these compounds. Thus, compounds according to formula I wherein L is CH₂-O, X is NH, Y is S, both A are NH and with the provision that at least one of R¹ and R² is not H have not been suggested for any medical use. Moreover, the disclosed thiourea compounds were not associated with the use in treatment of inflammatory diseases.

In another aspect the invention provides a compound according to formula I 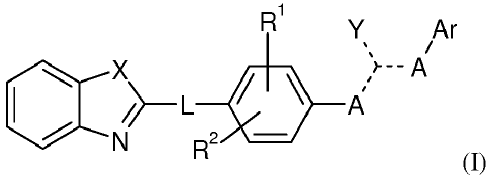
wherein L is CH₂-O, CH₂-S, CH₂-CH₂, or CH=CH;
R¹ and R² are independently selected out of H, halogen, C1 to C3 alkyl, halogenated C1 to C3 alkyl, C1 to C3 alkyloxy, or halogenated C1 to C3 alkyloxy;
Ar is an aryl moiety, preferably phenyl or naphthyl, or a moncyclic- or bicyclic heteroaryl moiety, wherein optionally the aryl or heteroaryl moiety is further substituted;
X is O, S, or NR³, wherein R³ is H, C1 to C3 alkyl, halogenated C1 to C3 alkyl, or *tert*-butyloxycarbonyl;
Y is O, S, NR⁴ or N(R⁴)R⁵, wherein R⁴ and R⁵ are independently selected out of H or C1 to C3 alkyl; and
wherein if Y is O, S, or NR⁴, then both A are NH, and the punctuated bonds represent a double bond to Y and single bonds to each A; or
wherein if Y is N(R⁴)R⁵, then one A is N, the other A is NH, and the punctuated bonds represent a double bond to the A being N, a single bond to the A being NH and a single bond to Y,
for use in treatment or prevention of an inflammatory disease or an inflammation-related disorder.

Preferably, the invention relates to a compound for use in treatment or prevention of conditions associated with a disorder of the immune system, like e.g. an inflammatory disease or an inflammation-related disorder. An inflammatory disease may be rheumatoid arthritis, asthma, allergic rhinitis, atherosclerosis, Alzheimer's disease or Parkinson's disease, preferably asthma. Moreover, the compounds may also be beneficial in treatment of an inflammation-related disorder, i.e. inflammation-related symptoms such as pain or swelling or inflammation-related disorders such as cardiovascular disease, diabetes, cancer, dermatitis and the like. Especially in case of asthma, reduction of leukotrienes is considered as mechanism to provide the therapeutic effect of established FLAP or 5-LOX inhibitors. The compound I-12 showed a similar effect regarding leukotriene level in an *in vivo* model as known compounds. Thus, the compounds according to the invention are preferably for use in treatment of prevention of asthma.

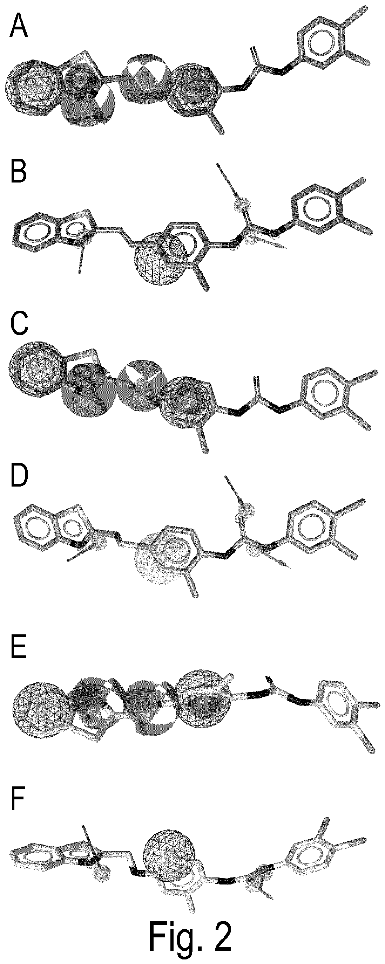

In view of the linker L, different embodiments were investigated. Preferably, the linker provides a spacer of two atoms, i.e. three bond lengths of double or single bonds separate the heteroaromate substructure from the central phenyl ring. Generally, compounds with an oxygen atom in the linker, i.e. an aryl ether function, showed good activity (e.g. I-12, EZ14) against both sEH and FLAP. Thus, CH₂-O may be preferred for L. However, also compounds wherein L is CH₂-CH₂ showed promising activity. For example L7, a compound wherein L is a C2 bridge (CH₂-CH₂), was shown to be a dual inhibitor of FLAP and sEH. Besides biological consideration, L is CH₂-O may be preferred as a convenient synthesis route for these diaryl(thio)urea compounds or diarylguanidine compounds is provided (s. below Examples 7 to 10). For the other potential linkers (L is CH₂-S, CH₂-CH₂ or CH=CH) theoretical synthesis routes are given (Example 11). Compounds wherein L is CH₂-S or CH=CH did also show a good fit with the pharmacophore models that were used when identifying I-12 (Fig. 2). Thus, L may be CH₂-S or CH=CH. For example a compound according to formula I may be *N*-[4-((benzo[d]thiazol-2-ylmethyl)thio)-2-methylphenyl[-*N*'-(3,4-dichlorophenyl)urea or *N*-[4-(2-(benzo[d]thiazol-2-yl)vinyl)-2-methylphenyl]-*N'*-(3,4-dichlorophenyl)urea.

In a preferred embodiment of the invention, L is CH₂-O or CH₂-CH₂.

In another aspect the invention relates to a compound according to formula II 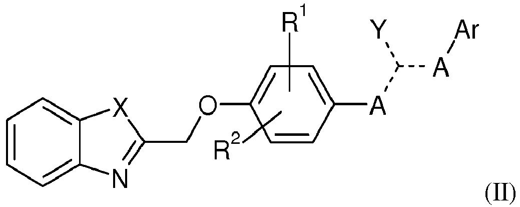
wherein R¹ and R² are independently selected out of H, halogen, C1 to C3 alkyl, halogenated C1 to C3 alkyl, C1 to C3 alkyloxy, or halogenated C1 to C3 alkyloxy;
Ar is an aryl moiety, preferably phenyl or naphthyl, or a moncyclic- or bicyclic heteroaryl moiety, wherein optionally the aryl or heteroaryl moiety is further substituted;
X is O or NR³, wherein R³ is H, C1 to C3 alkyl, halogenated C1 to C3 alkyl, or *tert*-butyloxycarbonyl;
Y is O, S, NR⁴ or N(R⁴)R⁵, wherein R⁴ and R⁵ are independently selected out of H or C1 to C3 alkyl; and
wherein if Y is O, S, or NR⁴, then both A are NH, and the punctuated bonds represent a double bond to Y and single bonds to each A; or
wherein if Y is N(R⁴)R⁵, then one A is N, the other A is NH, and the punctuated bonds represent a double bond to the A being N, a single bond to the A being NH and a single bond to Y;
with the provision that a compound in which L is CH₂-O, X is NH, Y is S, both A are NH and both R¹ and R² are H is excluded.

As commercial availability of compounds according to formula I is limited, new derivatives were synthesized. A new synthesis route was described for compounds according to formula II. One example of a compound according to formula II is the benzimidazole derivative of I-12, *N*-[4-(1*H*-Benzimidazol-2-ylmethoxy)-2-methylphenyl]-*N'*-(3,4-dichlorophenyl)-urea (Code: EZ14):

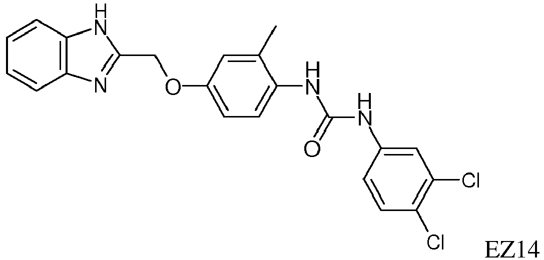

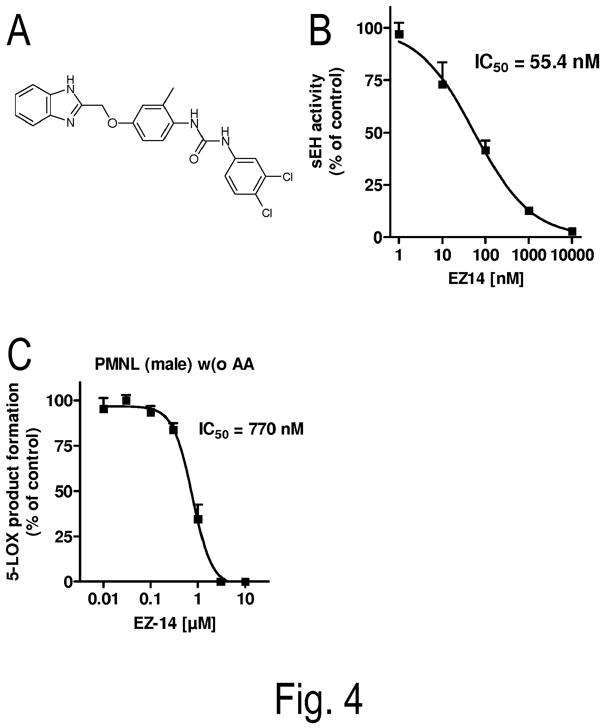

This compound showed similar effects on sEH activity and 5-LOX product formation as I-12 (Figure 4).

In a compound according to general formula II, the different residues individually may have the following preferred embodiments:
- Ar is an aryl moiety, preferably phenyl or naphthyl, more preferably phenyl, wherein optionally the aryl moiety is further substituted,
- R¹ and R² are independently selected out of H, halogen, C1 to C3 alkyl, halogenated C1 to C3 alkyl, C1 to C3 alkyloxy, or halogenated C1 to C3 alkyloxy, for example H or C1 to C3 alkyl,
- X is NR³, wherein R³ is H, C1 to C3 alkyl, halogenated C1 to C3 alkyl, or *tert*-butyloxycarbonyl, for example H or *tert*-butyloxycarbonyl,
- Y is O, S, NR⁴ or N(R⁴)R⁵, wherein R⁴ and R⁵ are independently H, C1 to C3 alkyl, and wherein C3 alkyl includes cyclopropyl.

The benzo-fused heterocycle forming part of the generic formula I may be benzoxazole, benzothiazole, or benzimidazole, i.e. X may be O, S or NR³. In a compound according to the invention of formula II, the benzo-fused heterocycle may be benzoxazole, or benzimidazole, i.e. X is O or NR³. Preferably, X is NR³ or S in a compound for use or according to the invention. Preferably, X is NR³ in a compound according to the invention.

The comparable activity of I-12 and EZ14 showed that benzothiazol-2-yl as well as 1H-benzimidazol-2-yl substitutions yield compounds with dual inhibition profile and especially low IC₅₀ for sEH. Benzimidazole compounds may be beneficial as they allow further modifications. For example if R³ is different from hydrogen further interactions within the interaction partner may be explored or the variation may allow to modulate physico-chemical properties.

R³ is defined to be selected out of the group consisting of H, C1 to C3 alkyl, halogenated C1 to C3 alkyl, or *tert*-butyloxycarbonyl. In course of this invention, C1 to C3 alkyl should be understood as any alkyl moieties with 1 to 3 carbon atoms, i.e. methyl, ethyl, prop-1-yl, prop-2-yl or cyclopropyl. Halogenated C1 to C3 alkyl refer to alkyl moieties with 1 to 3 carbon atoms, wherein at least one and up to all hydrogen atoms of the related C1 to C3 alkyl moiety is/are replaced by a halogen atom selected out of F, Cl, Br, or I, preferably F or Cl. An example for a halogenated C1 to C3 alkyl may be as simple as trifluorormethyl, but also 2-chloro-1,1,2-trifluoroethyl.

In the following, preferred embodiments of the invention or definition of residues may relate to a compound according to the invention as well as a compound for use according to the invention.

Regarding the aromatic group Ar, it may in general be any aromatic structure including monocyclic or polycyclic, e.g. bicyclic, moieties as well as heteroaryl moieties, which are substituted or unsubstituted. Aromatic carbocycles like phenyl or naphthyl may be considered preferred because compounds including these embodiments were shown to exhibit the desired properties. However, Ar may also be a heteroaryl moiety such as a five-membered or six-membered aromatic ring containing at least one N, O, or S atom, e.g. pyridyl, furyl, or thienyl.

In another preferred embodiment Ar is a phenyl group. Optionally, the phenyl is substituted with one or more substitutent(s). These substitutent(s) may be selected for example out of the group consisting of halogen, amino, C1 to C3 alkyl, halogenated C1 to C3 alkyl, C1 to C3 alkyloxy, or halogenated C1 to C3 alkyloxy, preferably halogen or halogenated C1 to C3 alkyl. Halogenated refers to moieties comprising at least one halogen atom selected out of F, Cl, Br, or I, e.g. comprising F, Cl or CF₃. For example, Ar is selected out of the group consisting of phenyl, 3,4-dichlorophenyl, 3,5-dichlorophenyl, 2-chlorophenyl, 3-chlorophenyl, 4-chlorophenyl, 3-trifluoromethylphenyl, 2-/3-/4-fluorophenyl, 2-/3-/4-methoxyphenyl, 2-/3-/4-aminophenyl or o-/m-/p-tolyl.

It was found that compounds, wherein the aryl moiety was not substituted (L3) or substituted with methoxy (L5 or L1), showed less inhibition on sEH activity compared to compounds wherein the aryl function was substituted with electron withdrawing groups, e.g. halogen or halogenated C1 to C3 alkyl. Furthermore, Ar is 3,4-dichlorophenyl in the best characterized compounds I-12 and EZ14.

Regarding the central phenyl ring, the two residues R¹ and R² may define up to two non-hydrogen substitutions. In case wherein R¹ and R² are both selected out of halogen, C1 to C3 alkyl, halogenated C1 to C3 alkyl, C1 to C3 alkyloxy, or halogenated C1 to C3 alkyloxy. For example the compounds for use according to formula I may be configured as follows: 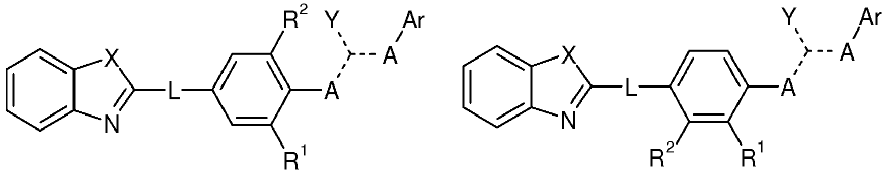 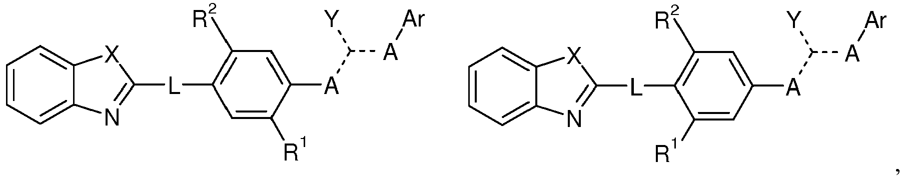 whereby the same substitution pattern may apply for a compound according to formula II.

In a preferred embodiment of the invention at least one of R¹ and R² is not hydrogen.

In one embodiment, only one of R¹ and R² is a not hydrogen, i.e. R² is hydrogen and R¹ is selected out of halogen, C1 to C3 alkyl, halogenated C1 to C3 alkyl, C1 to C3 alkyloxy, or halogenated C1 to C3 alkyloxy. In these cases, the compounds for use according to formula I may be configured as follows: 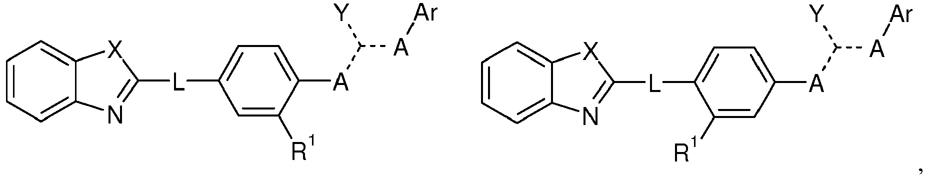 whereby the same substitution pattern may apply for a compound according to formula II.

In a preferred embodiment, R¹ is selected out of halogen, C1 to C3 alkyl, halogenated C1 to C3 alkyl, C1 to C3 alkyloxy, or halogenated C1 to C3 alkyloxy and R¹ is configured in *ortho-*position to the nitrogen, i.e. a compound according to formula III or IV:

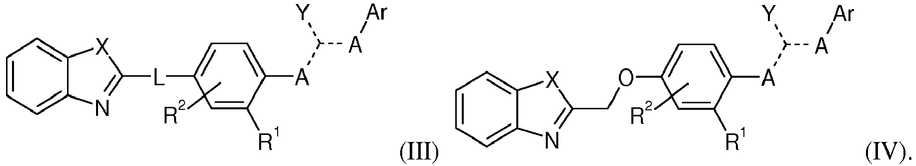

In the compounds L1 to L8 both R¹ and R² are hydrogen atoms and besides L7, those compounds did not show FLAP inhibition at the measured concentration. Therefore, those compounds, wherein at least one of R¹ and R² is not hydrogen, preferably wherein R¹ is not hydrogen, are preferred regarding anti-FLAP activity. When comparing EZ14 and EZ19, which differ to each other in that R¹ in *ortho*-position is methyl in EZ14 and hydrogen in EZ19, it is observed that the methyl group enhances anti-sEH activity. This structure-activity relation may be related to an influence of this moiety on the conformational distribution and therefore, methyl and other small substituents are preferred over hydrogen for R¹, like, e.g. C1 to C3 alkyl or halogenated C1 to C3 alkyl, preferably methyl.

In regard of the Y in formula I to IV, Y is selected out of O, S or N, i.e. the compounds being urea, thiourea or guanidino compounds, respectively. Urea, thiourea or guanidino compounds are characterized by a double bond, wherein the electrons of the double bond may be delocalized and may form part of a conjugated system with the adjacent aromatic substructures. Urea, thiourea as well as guanidine substructures may form tautomers. A person skilled in the art will consider resonance structures and tautomeric structures of a compound according to the invention as covered by the given Lewis formula. Preferably, Y is O or S, and more preferably Y is O. The invention provides compounds with urea motive, wherein Y is O and both B are NH. Classically a double bond is drawn or written for the carbon-oxygen bond, although the person skilled in the art is aware of the partial double bond character of the carbon-nitrogen bonds.

Urea compounds according to formula I and II, i.e., 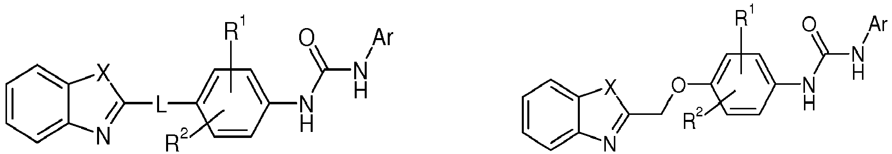 were shown to exhibit the desirable dual activity to a greater extent and thus, are preferred.

However, also thiourea compounds according to formula I and II, i.e. 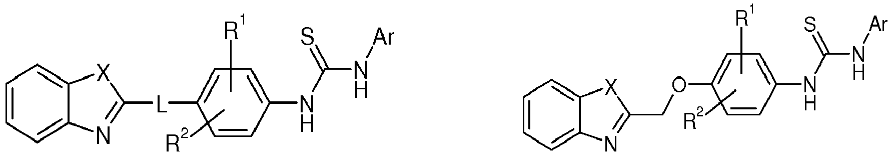 showed an inhibitory effect on 5-LOX product formation and sEH activity in a reasonable concentration.

Thus, in a preferred embodiment compounds for use according to the invention Y is O or S, and both A are NH, and the punctuated bonds represent a double bond to Y and single bonds to each A. Accordingly those preferred compounds are described by formula V or formula VI 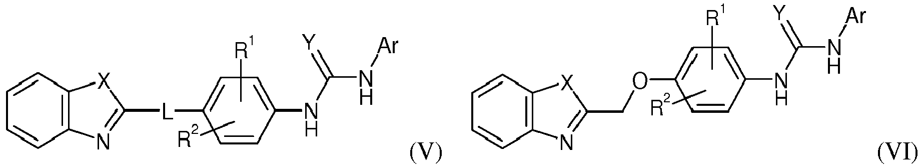 wherein Y is O or S, preferably O.

Alternatively to urea and thiourea compounds, the inventors synthesized guanidino compounds, wherein Y is N. This third nitrogen in the guanidino function may be further substituted and the inventors synthesized derivatives, wherein said nitrogen was substituted with one or two moieties. Thus, Y is NR⁴ or N(R⁴)R⁵. In guanidino compounds the delocalized double bond may generally be considered equally distributed between the three nitrogen atoms. Due to tautomerization and protonation in aqueous solution, the position of the hydrogen atoms and the double bond is often not properly reflected by a single Lewis formula. If Y is N(R⁴)R⁵, then one A is N and the other A is NH in formula I or II. In case of guanidino group with five substituents, the double bond is classically drawn to one the nitrogen atoms with a single substitution. Thus, the punctuated bonds are written as a double bond to the A being N, as a single bond to the A being NH and a single bond to Y. However, a person skilled in the art will understand that such a formula also covers the equivalent tautomers and resonance structures. The different variants of formula I and II may be written as follows: 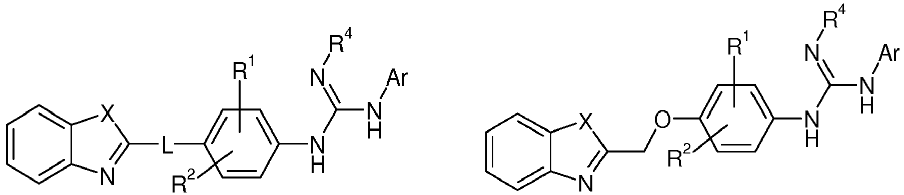 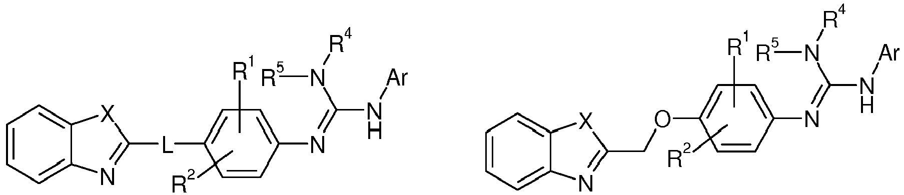 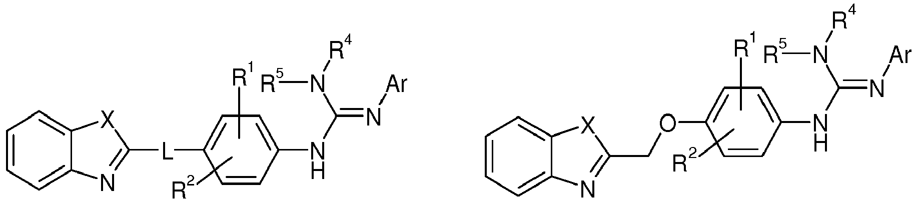 including embodiments, wherein R⁵ is hydrogen:

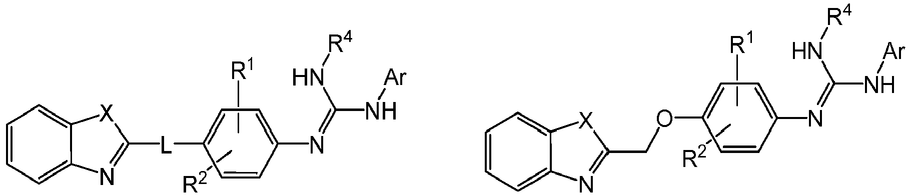

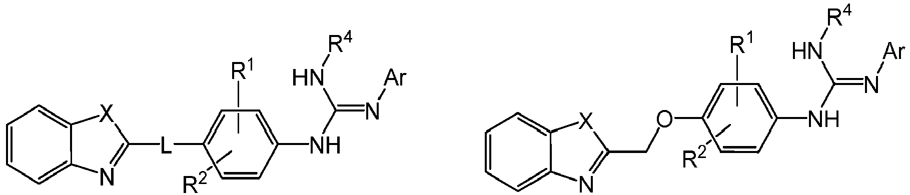

Guanidino compounds may be suitable for use according to the present invention especially because of their pharmacokinetic properties. The guanidino substructure is known to be basic, i.e. protonated in solution and may form salts. This polar function may increase solubility in compounds according to the invention while the activity may be retained. Compounds with guanidino substructure largely reproduced the same binding hypotheses comparable to the urea and thiourea compounds according to the invention (Fig. 2E and F, Example 1). Moreover, the inventors could show the successful syntheses of guanidino compounds according to formula II (Example 10).

In another preferred embodiment, the invention provides a compound or a compound for use according to the invention, wherein the compound is capable of inhibiting biochemically evaluated activity of sEH with concentrations of 0.1 µM or lower and 5-LOX product formation with concentrations of 1 µM or lower.

Such properties may be determined by a person skilled in the art with appropriate activity assays, said assays being previously disclosed or described in the detailed description below. Inhibition of sEH and 5-LOX product formation can for example be assayed as described below in Examples 2 and 3. Preferred compounds according to the invention with these desirable capabilities are for example EZ14 and I-12. Compounds showing the desired properties of inhibiting both sEH and 5-LOX at low concentrations are preferred. Compounds for use according to formula I or a compound according to formula II with at least one of the desired properties may also fall under the term "active compound" according to the present inventions.

A compound or a compound for use according to the present invention of the present invention may be provided as a medicament in the form of a pharmaceutical composition. The present invention provides a pharmaceutical composition comprising a compound of the present invention in association with at least one pharmaceutically acceptable excipient, e.g. appropriate carrier and/or diluent, e.g. including fillers, binders, disintegrants, flow conditioners, lubricants, sugars or sweeteners, fragrances, preservatives, stabilizers, wetting agents and/or emulsifiers, solubilizers, salts for regulating osmotic pressure and/or buffers.

A pharmaceutical composition according to the present invention may be used for treatment or prevention of an inflammatory disease or an inflammation-related disorder, preferably an inflammatory disease.

An active compound or composition according to the present invention may be administered by any conventional route, for example, nasal, buccal, rectal, oral administration (enterally); parenterally, e.g. including intravenous, intraarterial, intramuscular, intracardiac, subcutanous, transdermal (diffusion through the intact skin), transmucosal (diffusion through a mucous membrane), inhalational administration; e.g. in form of coated or uncoated tablets, capsules, (injectable) solutions, solid solutions, suspensions, dispersions, solid dispersions; e.g. in the form of ampoules, vials, in the form of inhaler powder, drops, sprays, or in the form of suppositories. In treatment of asthma or an inflammatory disease related to the respiratory tract administration via inhalation may be preferred.

An active compound of the present invention may be present in the composition, i.e. administered, in the form of a pharmaceutically acceptable salt, or in free form; optionally in the form of a solvate. A compound of the present invention in the form of a salt and/or in the form of a solvate exhibits the same order of activity as a compound of the present invention in free form.

An active compound of the present invention may be used for any method or use as described herein alone or in combination with one or more, at least one, other drug substance.

The invention also relates to a method for treatment or prevention, e.g. treatment or prevention of an inflammatory disease or an inflammation-related disorder, wherein one of the above described compound or compound for use or composition is administered to a subject in need thereof. The subject preferably is a human being.

In another aspect the invention relates to a process for synthesis of a compound according to formula II 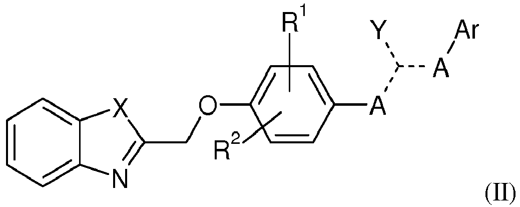
wherein R¹ and R² are independently selected out of H, halogen, C1 to C3 alkyl, halogenated C1 to C3 alkyl, C1 to C3 alkyloxy, or halogenated C1 to C3 alkyloxy;
Ar is an aryl moiety, preferably phenyl or naphthyl, or a moncyclic- or bicyclic heteroaryl moiety, wherein optionally the aryl or heteroaryl moiety is further substituted;
X is O, S, or NR³, wherein R³ is H, C1 to C3 alkyl, halogenated C1 to C3 alkyl, or *tert*-butyloxycarbonyl, preferably X is O or NR³;
Y is O, S, NR⁴ or N(R⁴)R⁵, wherein R⁴ and R⁵ are independently selected out of H or C1 to C3 alkyl; and
wherein if Y is O, S, or NR⁴, then both A are NH, and the punctuated bonds represent a double bond to Y and single bonds to each A; or
wherein if Y is N(R⁴)R⁵, then one A is N, the other A is NH, and the punctuated bonds represent a double bond to the A being N, a single bond to the A being NH and a single bond to Y;
comprising the steps of
a) providing a mixture of a compound according to formula VII 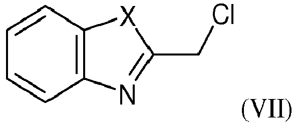 and a compound according to formula VIII 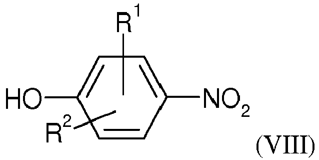 in conditions that allow them to form an ether and obtain the ether according to formula IX 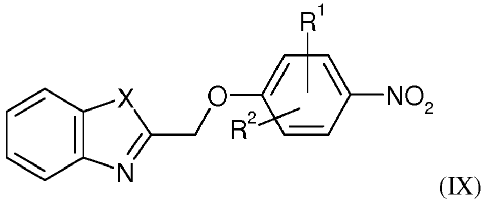
   wherein X is O, S, or NR³, wherein R³ is C1 to C3 alkyl, halogenated C1 to C3 alkyl, *tert*-butyloxycarbonyl or a protecting group;
   R¹ and R² are independently selected out of H, halogen, C1 to C3 alkyl, halogenated C1 to C3 alkyl, C1 to C3 alkyloxy, or halogenated C1 to C3 alkyloxy,
b) treating the compound according to formula IX obtained in step b) with molecular hydrogen or a source for molecular hydrogen and a metal catalyst under conditions that allow reduction of the compound according to formula IX to obtain an amino compound according to formula X 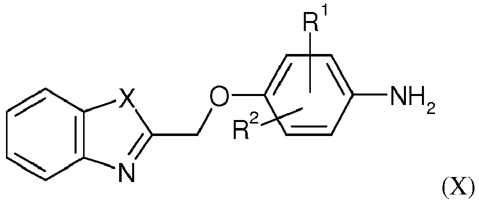
   wherein X is O, S, or NR³, wherein R³ is C1 to C3 alkyl, halogenated C1 to C3 alkyl, *tert*-butyloxycarbonyl or a protecting group;
   R¹ and R² are independently selected out of H, halogen, C1 to C3 alkyl, halogenated C1 to C3 alkyl, C1 to C3 alkyloxy, or halogenated C1 to C3 alkyloxy,
c) treating the amino compound according to formula X obtained in step b) with a compound according to formula XI 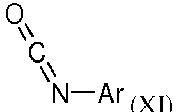 or formula XII 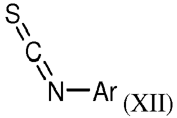 under conditions that a allow formation of a compound according to formula VI 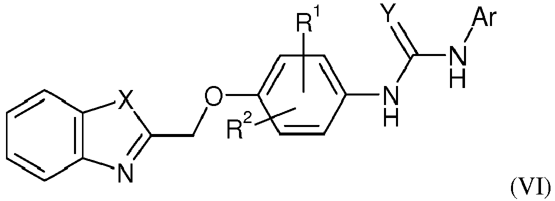
   wherein Y is O, when the amino compound is treated with a compound according to formula XI and Y is S, when the amino compound is treated with a compound according to formula XII;
   wherein X is O, S, or NR³, wherein R³ is C1 to C3 alkyl, halogenated C1 to C3 alkyl, *tert*-butyloxycarbonyl or a protecting group;
   R¹ and R² are independently selected out of H, halogen, C1 to C3 alkyl, halogenated C1 to C3 alkyl, C1 to C3 alkyloxy, or halogenated C1 to C3 alkyloxy; and
   Ar is an aryl moiety, preferably a phenyl or naphthyl moiety, or a moncyclic or bicyclic heteroaryl group, wherein the aryl or heteroaryl group is optionally further substituted; and
d) optionally, removal of a protecting group from a compound according to formula VI, provided that X is NR³, wherein R³ is *tert*-butyloxycarbonyl or another protecting group, to obtain a compound according to formula XIII 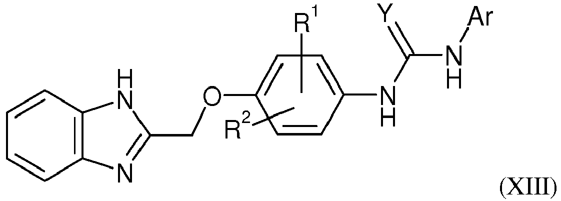
   wherein Y is O or S;
   R¹ and R² are independently selected out of H, halogen, C1 to C3 alkyl, halogenated C1 to C3 alkyl, C1 to C3 alkyloxy, or halogenated C1 to C3 alkyloxy; and
   Ar is an aryl moiety, preferably a phenyl or naphthyl moiety, or a moncyclic or bicyclic heteroaryl group, wherein the aryl or heteroaryl group is optionally further substituted.

The synthesis route according to the invention provides a mean to achieve compounds according to the invention, which were found to be useful as potential candidates for compounds for use according to the invention. In these examples, in formula VII, X is NR³, wherein R³ is *tert*-butyloxycarbonyl.

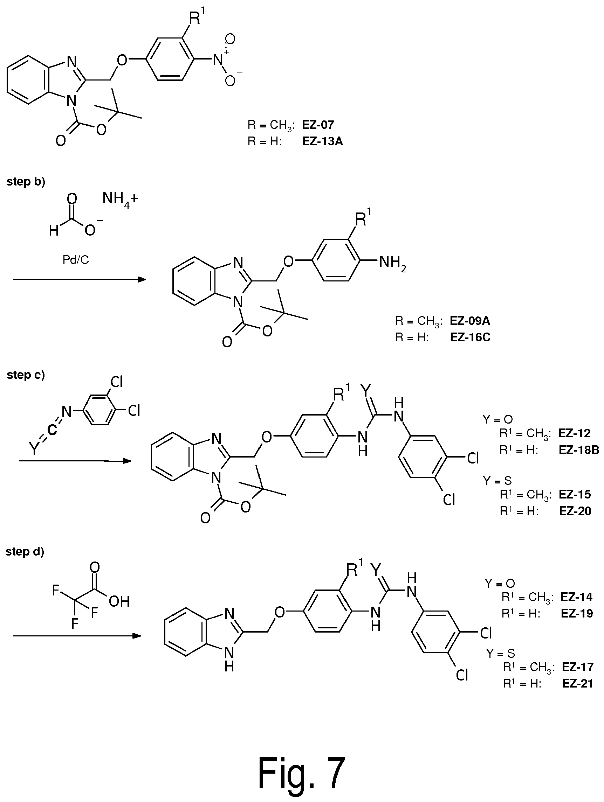

Exemplarily, a reaction scheme of the process for synthesis is shown in Figure 7. In this embodiment of the process according to the invention X is NR³, wherein R³ is C1 to C3 alkyl, halogenated C1 to C3 alkyl, tert-butyloxycarbonyl or a protecting group, preferably *tert-*butyloxycarbonyl. Preferably, R³ is a protecting group, more preferably tert-butoxycarbonyl, and in step d), the protecting group is removed by use of an acid (to obtain a compound according to formula XIII). The method is also suitable to provide compounds according to formula II, wherein X is S or O, i.e. benzthiazole or benzoxazole substructure as in Example 7 or Example 9.

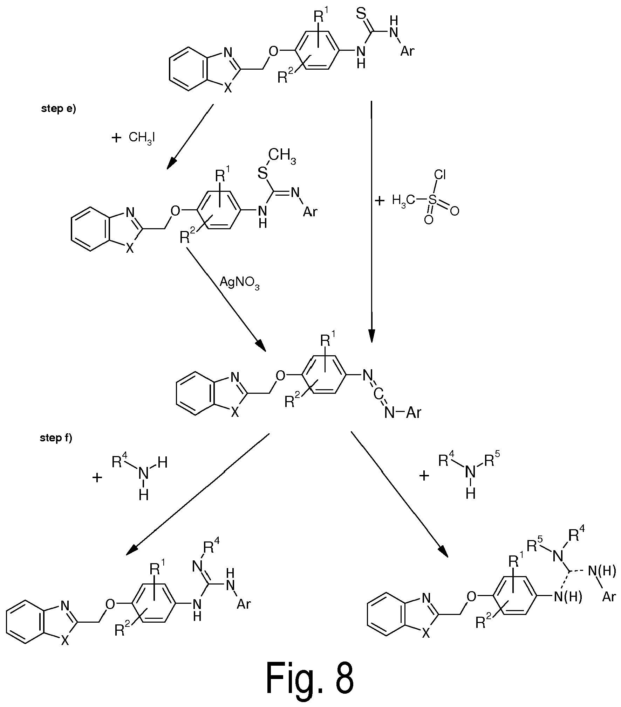

In an embodiment, the process is suitable for synthesis of a compound according to formula I, wherein Y is NR⁴ or N(R⁴)R⁵. For these guanidine compounds, the steps a) to c) are the same as for thiourea compounds. Subsequently, the thiourea compound may be transformed to a carbodiimide (step e), which may react with an amine to form a guanidine compound (step f). These reactions steps are illustrated in Figure 8, wherein two alternative routes are shown for step e) and wherein the product depends on the amine provided in step f).

In these embodiments, the process further comprises the steps of
e) treating the compound obtained in step c) with methyliodid to obtain a S-methylated isothiourea compound according to formula XIV 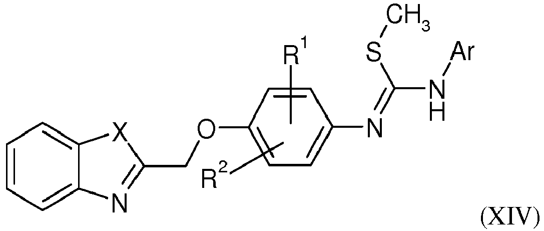
   wherein X, Ar, R¹ and R² are defined as above and subsequently treating the S-methylated thiourea compound with silver nitrate;
   or treating the compound obtained in step c) with methanesulfonyl chloride in presence of a base;
   wherein both alternative treatments (via S-methylated isothiourea or directly with methanesulfonyl chloride) results in a carbodiimide according to formula XV 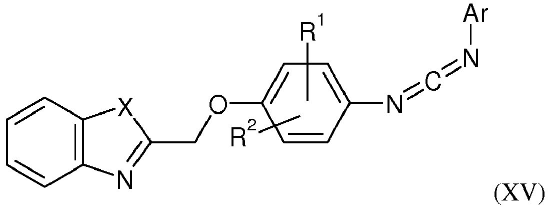 wherein X, Ar, R¹ and R² are defined as above, and
f) treating the carbodiimide obtained in step e) with an amine according to formula XVI 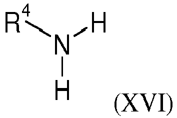 or an amine according to formula XVII 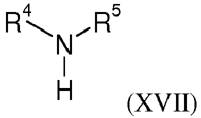 wherein R⁴ and R⁵ are independently selected out of H or C1 to C3 alkyl, preferably C1 to C3 alkyl;
   to obtain a compound according to formula II, 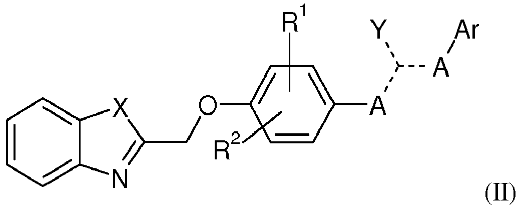
   wherein X, Ar, R¹ and R² are defined as above; and
   wherein when the carbodiimide compound was treated with an amine according to formula XVI, then is NR⁴, both A are NH, and the punctuated bonds represent a double bond to Y and single bonds to each A; or
   wherein when the carbodiimide compound was treated with an amine according to formula XVII, then Y is N(R⁴)R⁵, one A is N, the other A is NH, and the punctuated bonds represent a double bond to the A being N, a single bond to the A being NH and a single bond to Y.

Those further steps e) and f) are conducted after step c) and preferably, before the optional step d).

R⁴ or R⁵ may be hydrogen, thus, the amine according to formula XVI or XVII might also be ammonia. However, it is preferred that the amine is a secondary amine HN(R⁴)R⁵, to obtain a compound wherein Y is N(R⁴)R⁵ or the amine is a primary amine according H₂NR⁴, wherein R⁴ or R⁴ and R⁵ are independently selected out of C1 to C3 alkyl, halogenated C1 to C3 alkyl, preferably C1 to C3 alkyl, wherein C3 alkyl includes cycloalkyl. For example, an amine according to formula XVI may be cyclopropylamine and an amine according to formula XVII may be diethylamine.

Regarding the specific conditions and reactions disclosed in the examples, a person skilled in the art may consider varying the conditions for synthesis of the compounds according to his experience and may easily select alternatives while still following the process according to the invention. The following embodiments are considered as preferred and may independently be selected.
- step a) is performed in presence of a carbonate salt in an aprotic polar solvent and the mixture is heated at reflux,
- step b) is performed under inert atmosphere, and wherein the hydrogen source is for example ammonium formate and the metal catalyst is Pd/C, alternatively a hydrogenator might be applied in combination with the catalyst Raney nickel.

- step c) is conducted by providing the amino compound of step b) in an aprotic solvent and adding the compound of formula XI or XII dropwise at room temperature,
- step d) is conducted by removing the protecting group by use of an acid, if R³ is a protecting group, preferably *tert*-butoxycarbonyl.

Furthermore, in preferred embodiments of the process of the invention the starting materials are selected so that preferred embodiments of compounds or compounds for use as described above may be obtained. Preferably, the starting materials may be selected, wherein
- X is NR³ or O, preferably NR³, wherein R³ is a protecting group, preferably tert-butoxycarbonyl, in a compound according formula VII
- a compound according to formula VIII is 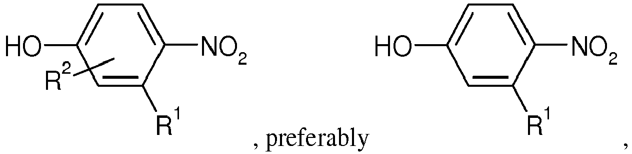 wherein R¹ is not hydrogen and selected from halogen, C1 to C3 alkyl, halogenated C1 to C3 alkyl, C1 to C3 alkyloxy, or halogenated C1 to C3 alkyloxy, more preferably R¹ is methyl.
- Ar in a compound according to formula XI or XII is a phenyl group, optionally the phenyl substituted with one or more of the moieties selected out of the group comprising F, Cl or CF₃, more preferably Ar is selected out the group comprising phenyl, 3,4-dichlorophen-1-yl, 2-chlorophen-1-yl, 3-chlorophen-1-yl, 4-chlorophen-1-yl, 3-trifluoromethylphen-1-yl.

### DETAILED DESCRIPTION OF THE INVENTION

The invention will now be described in more detail by the non-limiting examples.
Fig. 1 shows compound I-12 as virtual hit, wherein A) shows the alignment of I-12 to a ligand-based pharmacophore model of FLAP with two aromatic ring features (shown as ring with plane vector), two hydrophobic features (spheres), and two hydrogen bond acceptor features (partial spheres); the excluded volumes are not shown for clarity and B) shows the alignment of I-12 to a structure based sEH model with a hydrophobic feature (sphere), a hydrogen bond acceptor and a hydrogen bond donor (arrows), the surface of the binding pocket is shown as well as individual residues of the sEH structure.
Fig. 2 shows the pharmacophore fit of compounds according to formula II, for the FLAP model (A, C, E) described in Fig. 1A and the sEH model (B, D, F) described in Fig. 1B. Fig. 2A) and B) show a compound analogue to I-12, wherein L is CH=CH; C) and D) show a compound, wherein L is CH₂-S and in E) and F) a compound is shown wherein Y is NH.
Fig. 3 shows compound I-12 and its biological activity, wherein A) shows the compound's structure, B) shows the concentration-dependent inhibition of the sEH activity (n=3), C) the inhibition of 5-LOX product formation in human male leukocytes, and D) the inhibition of 5-LOX product formation in human male monocytes.
Fig. 4 shows compound EZ14 and its biological activity, wherein A) shows the compounds structure, B) shows the concentration-dependent inhibition of the sEH activity (n=3), C) the inhibition of 5-LOX product formation.
Fig. 5 shows further characterization of I-12 as FLAP inhibitor, wherein A) shows the concentration-dependent effect on the isolated 5-LOX enzyme, B) shows the concentration-dependent effect of I-12 on 5-LOX product formation of HEK293 cells without FLAP (closed square) in comparison to the presence of FLAP (open square), C) shows the concentration-dependent effect of I-12 on formation of the 15-LOX product 15-HETE in comparison to the positive control MK866 (a known FLAP inhibitor).
Fig. 6 shows preclinical characterization of I-12, wherein A) to D) shows cell viability of MM6 cells (A and B) and isolated human monocytes (C and D) after incubation with the indicated compound for 24 h (A and C) or 48 h (B and D).
Fig. 7 shows a reaction scheme for synthesis of (thio)urea compounds according to the general formula II, i.e. the steps b) to d) are shown for individual compounds as synthesized according to example 8.
Fig. 8 shows a reaction scheme for synthesis of guanidino compounds according to general formula II, wherein Y is NR⁴ or N(R⁴)R⁵ starting from a thiourea compound according to formula II, wherein Y is S.

### Example 1: Identification of hit compounds by virtual screening

3D-pharmacophore modeling and virtual screening were performed in LigandScout (http://www.inteligand.com/ligandscout/) and Discovery Studio (www.biovia.com).

Two ligand-based pharmacophore models were developed based on 11 FLAP inhibitors from literature. The models were designed to retrieve new potential FLAP inhibitors from large compound databases. The automatically generated models were optimized by removing features, altering feature sizes, and adding exclusion volume coats. In the end of the optimization process the two models were able to find all 11 testset compounds (known FLAP inhibitors used as positive control). Their selectivity was checked by screening them against the NCI database.

For identification of novel FLAP inhibitors, the SPECS library with 202 920 compounds was searched with both models independently to retrieve compounds that fit the models (virtual screening). 10 hits per model were selected for biological testing according to their fit-values.

For sEH, eight structure-based pharmacophore models were available (Waltenberger, B.; Garscha, U.; Temml, V.; Liers, J.; Werz, O.; Schuster, D.; Stuppner, H., Journal of Chemical Information and Modeling 2016, 56, 747-762, doi:10.1021/acs.jcim.5b00592). Seven of the 20 selected FLAP inhibitor candidates also matched at least one model for sEH. One of those compounds predicted as dual hit was I-12, for which Figure 1 shows the alignment with the pharmacophore model for FLAP and sEH.

Alternative compounds according to the invention have been investigated experimentally (s. below) or by *in silico* modeling. *N*-[4-((benzo[d]thiazol-2-ylmethyl)thio)-2-methylphenyl]-N'-(3,4-dichlorophenyl)urea and *N*-(4-(2-(benzo[d]thiazol-2-yl)vinyl)-2-methylphenyl)- N'-(3,4-dichlorophenyl)urea were investigated to characterize alternative linkers (Fig. 2A to D). The overall fit of those derivatives is very convenient, although certain features might be missed (i.e. one hydrogen bond feature in Fig. 2C). Thus, it may be expected that compounds wherein L is CH₂-S or CH=CH have a similar activity as I-12.

Also a N,N'-diaryl guanidine at the position of the urea or thiourea substructure fit the binding hypothesis represented by the models (Fig. 2E and 2F). Moreover, a protonated guanidinium motive might form a salt bridge with the aspartate in the sEH pocket (Asp332 in Fig. 1B). Accordingly, the invention includes compounds according to formula I, wherein Y is NR⁴ or N(R⁴)R⁵.

### Example 2: Measurement of 5-LOX product formation and FLAP inhibition

Biological testing for FLAP-mediated inhibition of 5-LOX product synthesis was conducted in two assays. Details of the assays were described previously (Pergola, C.; Gerstmeier, J.; Monch, B.; Caliskan, B.; Luderer, S.; Weinigel, C.; Barz, D.; Maczewsky, J.; Pace, S.; Rossi, A.; Sautebin, L.; Banoglu, E.; Werz, O., British Journal of Pharmacology 2014, 171 (12), 3051-64. doi: 10.1111/bph.12625)

Briefly, first a cell-based assay allows to determine the inhibition of the 5-LOX product biosynthesis. The cell suspension contained human neutrophils (PMNL) or monocytes, wherein the cells were isolated from buffy coats obtained from healthy donors pooled by gender. An aliquot cell suspension was incubated with the candidate compound for 15 min at 37 °C. The cells are treated with calcium ionophore A23187 to stimulate 5-LOX product formation for further 10 min (37 °C), optionally in presence of various concentrations of AA. The reaction was stopped with 1 mL methanol on ice.

The second assay is a FLAP-independent cell-free enzymatic assay testing for 5-LOX activity, in which second assay inactivity indicates FLAP as target. Recombinant 5-LOX enzyme is expressed in E. coli and purified by affinity chromatography. The enzyme was preincubated for 15 min with the potential inhibitor on ice and subsequently exposed to 2 mM CaCl₂ and 20 µM AA for 10 min at 37°C.

In both assays, product quantification was conducted with RP-HPLC and UV detection after solid phase extraction and addition of 200 ng prostaglandin B1 as internal standard.

### Example 3: Measurement of sEH activity

A detailed description of the assay has been described the inventors (Waltenberger B.; Garscha U.; Temml V.; Liers J.; Werz, O.; Schuster D.; Stuppner H. Discovery of potent soluble epoxide hydrolase (sEH) inhibitors by pharmacophore-based virtual screening. Journal of Chemical Information and Modeling 2016, 56, 747-762, doi:10.1021/acs.jcim.5b00592). In brief, sEH was expressed in transfected Sf9-cells. Isolation of sEH from the cell pellet was conducted with a benzylthiosepharose matrix. After purification, sEH enzyme activity was evaluated. sEH is provided in concentration of 0.27 to 1.35 µg/mL and supplied with 50 µM of the substrate PHOME (3-phenyl-cyano(6-methoxy-2-naphthalenyl)methyl ester-2-oxiraneacetic acid, Cayman Chemical (Biomol, Hamburg, DE)). The reaction was stopped with ZnSO₄ (0.2 M) after 60 min incubation. Hydrolysis of PHOME by sEH produces the highly fluorescent 6-methoxy-2-naphthaldehyde which was analyzed for fluorescence (Ext. 330 nm / Em. 465 nm, 37 °C). Inhibitors were tested for self fluorescence. Positive controls were AUDA (12-(3-adamantan-1-yl-ureido)dodecanacid) and DCU (N,N'-dicyclohexylurea) at 10 µM.

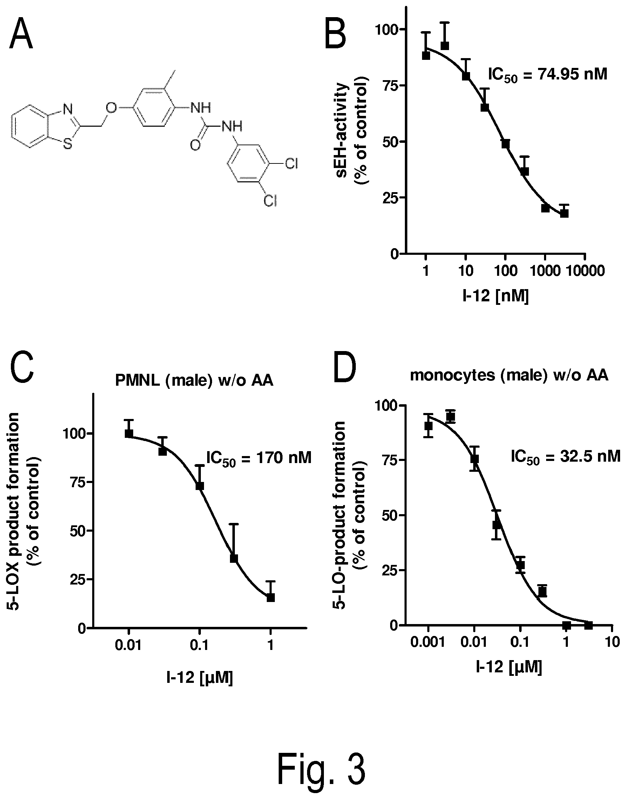

Results are shown in Figures 3 and 4, as well as Table 1. Effect on 5-LOX product formation and sEH activity is determined as described above. In case of observed effect on 5-LOX product formation in the cellular assay, 5-LOX activity was investigated with isolated 5-LOX. Absence of 5-LOX activity in the cell-free assay and presence of cellular inhibition of product formation indicates FLAP activity.

**Table 1: Summary of activity data for investigated compounds**

| Compound | Code | Effect on 5-LOX product formation in PMNL | Effect on isolated 5-LOX | Effect on FLAP activity | Effect on sEH activity |
|---|---|---|---|---|---|
| 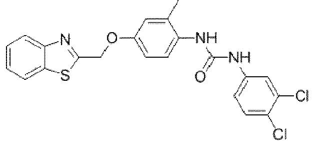 | I-12 | IC₅₀ = 240 nM | IC₅₀ > 10µM | active | IC₅₀ = 20 nM |
| 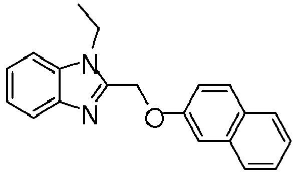 | I12-1* | inactive | no data | inactive | inactive |
| 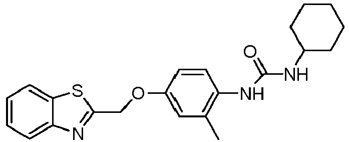 | I12-2* | inactive | no data | inactive | 75% inhibition at 0.1 µM |
| 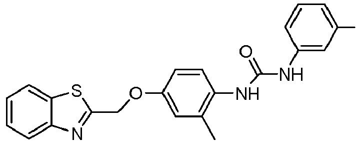 | I12-3 | 100% inhibition at 1 µM | no data | active | 55% inhibition at 0.1 µM |
| 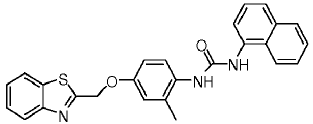 | I12-4 | 70% inhibition at 1 µM | no data | active | inactive |
| 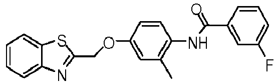 | I12-5* | inactive | no data | inactive | inactive |
| 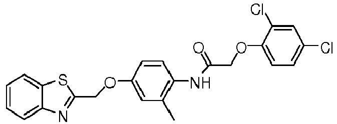 | I12-6* | inactive | no data | inactive | inactive |
| 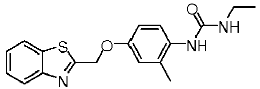 | I12-7* | inactive | no data | inactive | inactive |
| 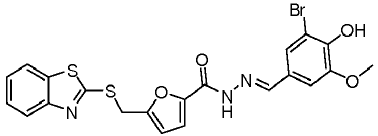 | I12-8* | inactive | no data | inactive | 30% inhibition at 0.1 µM |
| 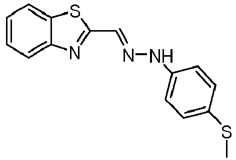 | I12-9* | inactive | no data | inactive | inactive |
| 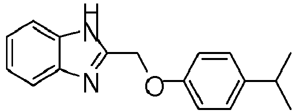 | I12-10* | inactive | no data | inactive | inactive |
| 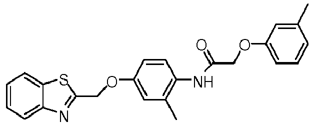 | I12-11* | 97% inhibition at 1µM | no data | active | inactive |
| 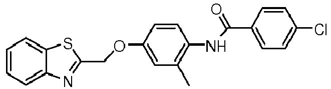 | I12-12* | inactive | no data | inactive | inactive |
| 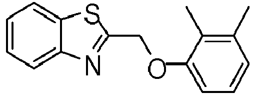 | I12-13* | inactive | no data | inactive | inactive |
| 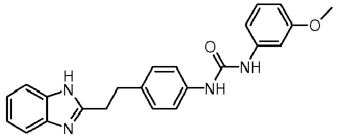 | L1 | inactive | no data | inactive | 30% inhibition at 0.1 µM |
| 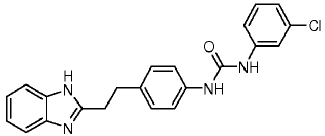 | L2 | inactive | no data | inactive | 50% inhibition at 0.1 µM |
| 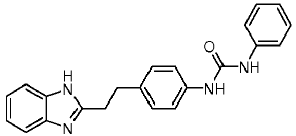 | L3 | inactive | no data | inactive | 30% inhibition at 0.1 µM |
| 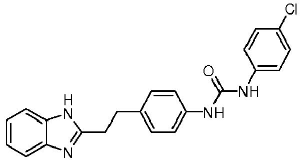 | L4 | inactive | no data | inactive | 78% inhibition at 0.1 µM |
| 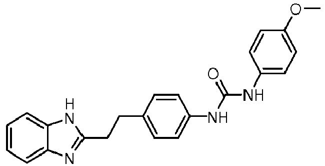 | L5 | inactive | no data | inactive | 50% inhibition at 0.1 µM |
| 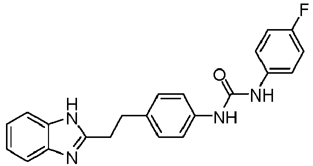 | L6 | inactive | no data | inactive | 55% inhibition at 0.1 µM |
| 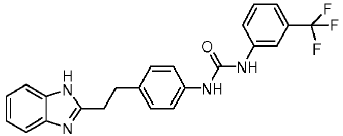 | L7 | 42% inhibition at 1µM | no data | active | 70% inhibition at 0.1 µM |
| 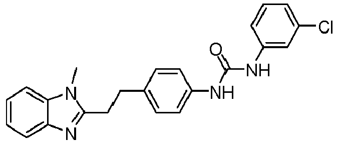 | L8 | inactive | no data | inactive | 65% inhibition at 0.1 µM |
| 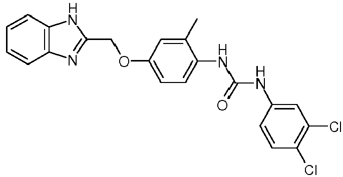 | EZ14 | IC₅₀ = 0.77 µM | no data | no data | IC₅₀ = 55 nM |
| 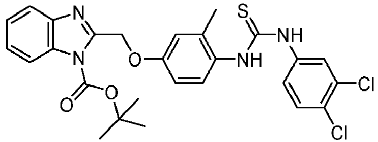 | EZ15 | ∼39% inhibition at 10 µM | ∼33% inhibitio n at 10 µM | inactive | ∼66 % inhibition at 10 µM |
| 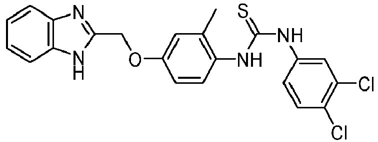 | EZ17 | IC₅₀ = 0.82 µM | 65% inhibitio n at 10 µM | active | ∼64% inhibition at 10 µM |
| 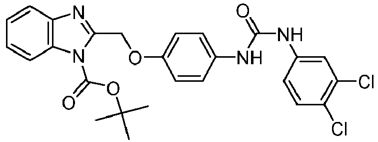 | EZ18 | ∼ 40 % inhibition at 10 µM | ∼22 % inhibitio n at 10 µM | no data | ∼67% inhibition at 0.1 µM |
| 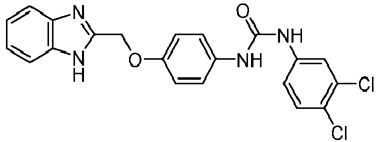 | EZ19 | IC₅₀ = 0.55 µM | ∼33 % inhibitio n at 10 µM | active | ∼52 % inhibition at 0.1 µM |
| 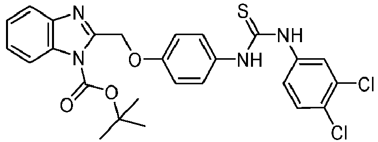 | EZ20 | -65 % inhibition at 10 µM | ∼39% inhibitio n at 10 µM | inactive | ∼39% inhibition at 0.1 µM |
| 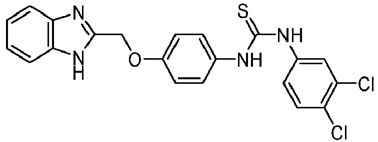 | EZ21 | IC₅₀ = 0.69 µM | 87 % inhibitio n at 10 µM | no data | ∼54% inhibition at 10 µM |
| 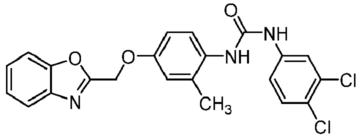 | SE04 | ∼85% inhibition at 1 µM | no data | no data | ∼87% inhibition at 1 µM |
| 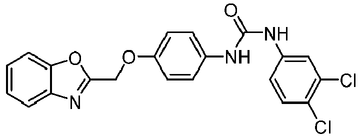 | TS04 | inactive | no data | inactive | ∼96% inhibition at 1 µM |

| | | | | | |
|---|---|---|---|---|---|
| * comparative example, not according to the invention. | | | | | |

### Example 4: Further biological characterization of I-12

For I-12, the effect of AA concentration on the inhibition of 5-LOX product inhibition (measurement as described above) was studied and showed that, upon provision of high AA concentration, the inhibitory effect of I-12 can be counteracted, as AA competes with FLAP inhibitors for binding to FLAP. Moreover, it should be noted that at concentrations of 60 µM AA, I-12 could not achieve an inhibition of product formation below 50 % even when higher concentrations of the inhibitor were use.

**Table 2: IC₅₀ values for 5-LOX product formation I-12 in male PMNL cells**

| Exogenous AA (µM) | IC₅₀ (µM) |
|---|---|
| 0 | 0.17 |
| 3 | 0.38 |
| 10 | 1.6 |
| 30 | 2.1 |
| 60 | 9.1 |

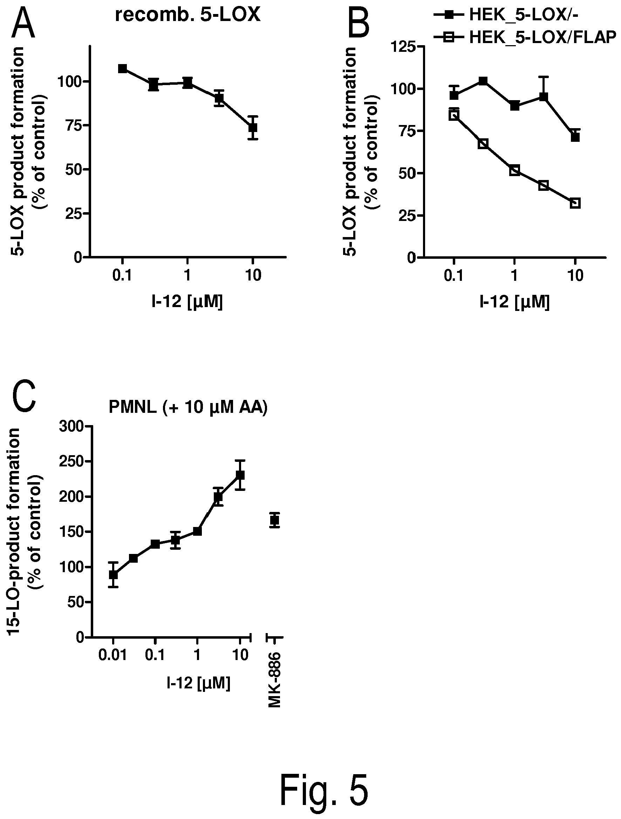

Furthermore, HEK cells without and with FLAP were compared, to proof the FLAP specific mechanism of I-12 (Jana Gerstmeier, Christina Weinigel, Dagmar Barz, Oliver Werz, Ulrike Garscha, An experimental cell-based model for studying the cell biology and molecular pharmacology of 5-lipoxygenase-activating protein in leukotriene biosynthesis, Biochimica et Biophysica Acta (BBA) - General Subjects, 1840(9), 2014, 2961-2969, DOI 10.1016/j.bbagen.2014.05.016). As shown in Figure 5B, I-12 has nearly no effect on 5-LOX product formation in HEK cells without FLAP. However, it inhibits the product formation in the cells that express FLAP.

Moreover, I-12 did not inhibit the product formation of 12-LOX nor 15-LOX in neutrophils tested with exogenous supply of AA in concentrations of 10, 30 or 60 µM (data not shown). For 15-LOX even an increase of the product formation was observed at 10 µM AA (Fig. 5C). This could be beneficial as the 15-LOX product 15-HETE is anti-inflammatory.

COX-1 and COX-2 activity and, similarly, mPGES are enzymes of the AA cascade, which were not affected by I-12 tested in concentrations up to 10 µM of the FLAP inhibitor (data not shown).

### Example 5: Cell viability

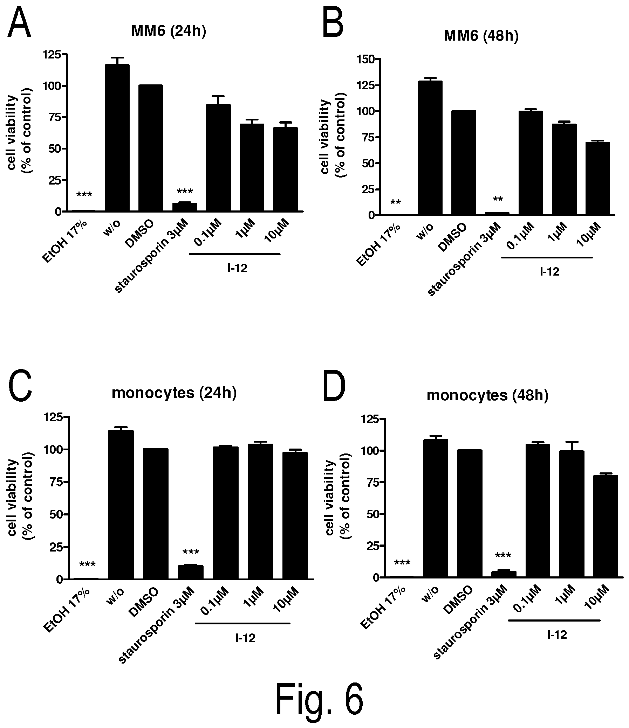

Cell viability under I-12 was examined by a MTT-assay. MM6 cells (a model cell line for human monocytes) and isolated human monocytes were investigated after incubation of different concentration of I-12 for 24 or 48 h. DMSO was used as reference and ethanol and staurosporin as positive control. Results are shown in Figures 6A to D.

### Example 6: Mouse model for induced peritonitis

Compound I-12 was evaluated *in vivo* in a peritonitis model in mice. The experimental protocols were approved by the Animal Care Committee of the University of Naples Federico II, in compliance with Italian regulations on protection of animals used for experimental and other scientific purpose (Ministerial Decree 116/92) as well as with the European Economic Community regulations (Official Journal of E.C. L 358/1 12/18/1986); protocol number: 2014/0018760 27^{th} February 2014. CD1 male mice with an age of six to eight weeks were investigated. The leukotriene mediated peritonitis is induced by injection of 1 mg zymosan in 50 µl saline solution. 30 min prior to induction, the candidate compound was applied as intraperitoneal injection in different concentrations as well as DMSO as negative control and positive controls (MK886, zileuton). Results are given in Table 3, wherein LTC3 and LTB 4 levels are measured as well as vascular permeability (VP) and the cell recruitment in the peritoneal cavity (Cell Rec).

**Table 3: Results from an in vivo study with a mouse peritonitis model**

| | LTC₄ (ng/ml) | LTB₄ (pg/ml) | VP (abs 610 nm) | Cell Rec (10⁶/ml) |
|---|---|---|---|---|
| Vehicle | 87.3±9.65 | 682±112 | 0.623±0.035 | 7.64±0.514 |
| | N= 17 | N=10 | N=5 | N=10 |
| I-12 1 mg | 26.0±9.47 | 280±42.8 | | 5.93±0.321 |
| | 70%*** | 59%** | | 22% |
| | N=7 | N=10 | | N=10 |
| I-12 3 mg | 30.5±10.3 | 345±69.2 | | 4.4±0.509 |
| | 66%*** | 50%* | | 42%*** |
| | N=6 | N=10 | | N=10 |
| I-12 10 mg | 25.8±2.66 | 193±34.4 | 0.347±0.09 | 3.36±0.439 |
| | 70%*** | 72%*** | 38% | 56%*** |
| | N=19 | N=10v | N=5 | N=10 |
| MK886 1 mg | 24.2±2.98 | 258±48 | | 4.54±0.326 |
| | 73%*** | 63%** | | 41%*** |
| | N=10 | N=10 | | N=10 |
| MK886 3 mg | | | 0.303±0.018 | |
| | | | 51% | |
| | | | N=5 | |
| ZIL 10 mg | 56.5±4.58 | | 0.639±0.104 | |
| | 36%* | | N=5 | |
| | N=12 | | | |

In Table 3, values in percent indicate the percent inhibition in respect to the vehicle control. Statistical significance is determined via ANOVA Bonferroni, wherein * indicates p<0.05; ** indicates p<0.01; *** indicates p<0.001 vs. vehicle. N indicates the number of investigated mice.

Already at concentrations of 1 mg, the inhibitor I-12 exhibited a similar reduction of LTC₄. The effect was similar to the established FLAP antagonist MK-886 and better than for 10 mg of the 5-LOX inhibitor zileuton.

### Example 7: Synthesis of I-12

I-12 was commercially available before (CAS Registry Number 724453-98-9), but the synthesis was not disclosed.

### Step a)

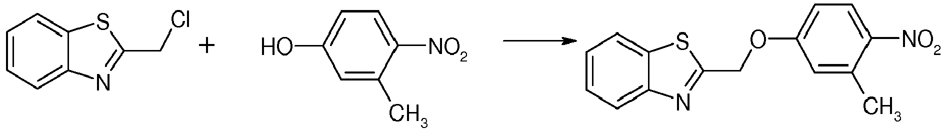

### 2-(3-Methyl-4-nitrophenoxymethyl)benzothiazole

The compound (CAS Registry Number 197364-74-2) was synthesized according to a procedure described in the literature (J. Med. Chem. 30/2, 400-405 (1987)). A mixture of 2-chloromethylbenzothiazole (3 mmol, 551 mg), of 3-methyl-4-nitrophenol (3 mmol, 459 mg), caesium carbonate (3 mmol, 977 mg), sodium carbonate (3 mmol, 318 mg), potassium iodide (1 mmol, 150 mg) and acetone (40 mL) was heated at reflux until the starting material was consumed as determined by tlc (ca. 6 h). The mixture was filtered and the resulting solution was partially concentrated *in vacuo.* The crystals thus formed were isolated, washed with acetone, and dried to give 663 mg (74 % yield) of the pure product as light orange crystals, mp 146-148 °C.
IR: no characteristic absorption bands. ¹H-NMR (DMSO-d₆) δ [ppm]: 8.15-8.00 (m, 3H, arene H), 7.58-7.42 (m, 2H, arene H), 7.23 (d, J = 2.8 Hz, 1H, arene H), 7.14 (dd, J = 9.0, 2.8 Hz, 1H, arene H), 5.74 (s, 2H, CH₂), 2.54 (s, 3H, CH₃).

### Step b)

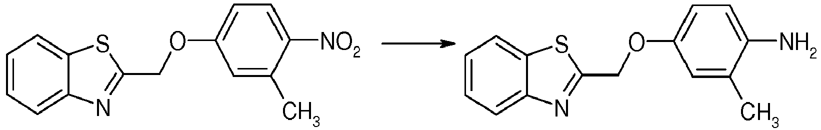

### 2-(4-Amino-3-methylphenoxymethyl)benzothiazole

A mixture of the nitro compound (918 mg, 3.06 mmol), in ethanol (250 mL) was reduced on a Parr hydrogenator at 40 psi using Raney nickel catalyst until the reduction was complete (determined by tlc, ca. 10 hrs.). Then, the mixture was filtered to remove the catalyst and the filtrate was evaporated under reduced pressure. The product thus obtained (CAS Registry number: 197364-75-3) was purified by column chromatography (silica gel, eluent: dichloromethane/ethyl acetate = 1/1) to give 720 mg (87 %) of desired product as a beige solid, mp 109-111 °C.
IR: 3424, 3296, 3177. ¹H-NMR (DMSO-d₆) δ [ppm]: 8.09 (dd, J = 7.6, 1.0 Hz, 1 H, arene H), 7.99 (dd, J = 7.0, 1.0 Hz, 1 H, arene H), 7.55-7.39 (m, 2H, arene H), 6.75-6.65 (m, 2H, arene H), 6.53 (d, J = 8.2 Hz, 1H, arene H), 5.41 (s, 2H, CH₂), 4.48 (s br, 2H, NH₂), 2.02 (s, 3H, CH₃).

### Step c)

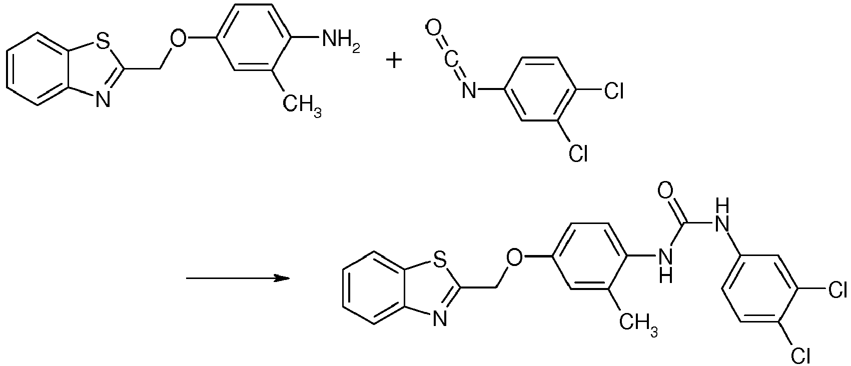

***N*-[4-(2-benzothiazolylmethoxy)-2-methylphenyl]-*N'*-(3,4-dichlorophenyl)-urea** (I-12) A solution of 3,4-dichlorophenylisocyanate (1.65 mmol, 310 mg) in dry tetrahydrofuran (2 *mL) was* added dropwise to a solution of the amino compound (1.50 mmol, 406 mg) in dry tetrahydrofuran (15 mL) and the resulting mixture was stirred at room temperature for 2 h. Then, diethyl ether was added and the solid obtained was collected, washed with tetrahydrofuran, and dried. The product was purified by treatment with acetone to yield 594 mg (86 %) of the product as a white solid, mp 253-253.5 °C.
IR [cm⁻¹] 3274, 1639. ¹H-NMR (DMSO-d₆) δ [ppm]: 9.84 (s, 1H, *D₂O exchangeable,* NH), 8.31 (s, 1H, *D₂O exchangeable,* NH), 8.13-8.09 ('m', 1H, arene H), 8.03-7.99 ('m', 1H, arene H), 7.87 (d, J = 2.2 Hz, 1H, arene H), 7.58-7.40 (m, 4H, arene H), 7.31 (dd, J = 8.9, 2.5 Hz, 1H, arene H), 6.97-6.86 (m, 2H, arene H), 5.55 (s, 2H, CH₂), 2.22 (s, 3H, CH₃). Calcd for C₂₂H₁₇Cl₂N₃O₂S: C, 57.65, H, 3.74; N, 9.17; S, 7.00. Found: C, 57.63, H, 3.72, N, 9.21; S, 6.93.

### Example 8: Synthesis of benzimidazole compounds according to formula II

In the following, syntheses of four compounds according to the generic formula II (EZ14, EZ19, EZ17 and EZ21), wherein X is NR³ are described and intermediate products characterized. In some cases alternative conditions are described. The overall reaction scheme for steps b) to d) is also exemplarily given in Figure 7.

### Step a)

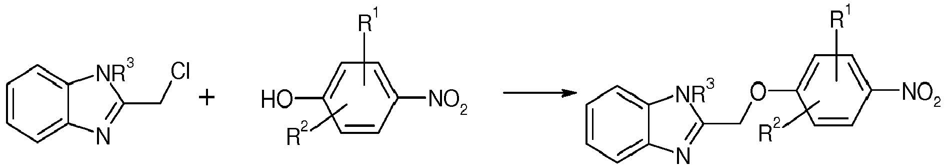

### 1-tert.-Butoxycarbonyl-2-[(3-methyl-4-nitrophenoxy)-methyl]-1H-benzimidazole (EZ07D)

A mixture of 0.368 g (2.4 mmol, 1.2 equiv.) of 3-methyl-4-nitrophenol and 0.415 g (3 mmol, 1.5 equiv.) of potassium carbonate in 20 mL of dry acetone was stirred for 1 hour. Then, 0.533 g (2 mmol, 1 equiv.) of 1-*tert*-butoxycarbonyl-2-chloromethyl-1*H*-benzimidazole were added and the reaction mixture was heated at reflux until the starting material was consumed as determined by tlc (ca. 9 h). Then, the solvent was removed by distillation under reduced pressure, the residue was treated with water and extracted with dichloromethane. The organic phase was washed with 2N sodium hydroxide solution, water and brine, and dried over anhydrous sodium sulfate. After removing the solvent, the raw product (0.753 g (98.2 %), orange solid) was treated with ethyl acetate to give 0.296 g (39 %) of the pure product as light yellow crystals. The filtrate was further purified by column chromatography (silica gel, eluent: ethyl acetate) to give additional 0.248 g (32 %) of the desired pure product (total yield: 71 %). Mp 152-154° C. IR [cm⁻¹]: 1735 (C=O). ¹H-NMR (DMSO-d₆) δ [ppm]: 1.61 (s, 9H, (CH₃)₃), 2.54 (s, 3H, CH₃), 5.65 (s, 2H, CH₂), 7.07-7.17 (m, 2H, arene H), 7.31-7.46 (m, 2H, arene H), 7.67-7.71 (m, 1H, arene H), 7.95-8.07 (m, 2H, arene H).

### 1-tert.-Butoxycarbonyl-2-[(4-nitrophenoxy)-methyl]-1H-benzimidazoles

Generally and alternative to above given procedure step a) can be conducted as follows: A suspension of 1.2 equivalents of the appropriate 4-nitrophenol, 1.5 equivalents of potassium carbonate, and 1 equivalent of 1-*tert*-butoxycarbonyl-2-chloromethyl-1*H*-benzimidazole in dry acetone (ca 1.5 mL/mmol) was heated in a closed 10 mL microwave reaction vial containing a magnetic stirring bar using a CEM Discover SP microwave synthesizer (2.45 GHz) for 2-3 h to 60-70 °C. Then, the mixture was treated with water and extracted with dichloromethane. The organic phase was washed with 2N sodium hydroxide solution, water and brine, and dried over anhydrous sodium sulfate. After removing the solvent, the raw product was purified as described below.

### 1-tert.-Butoxycarbonyl-2-[(3-methyl-4-nitrophenoxy)-methyl]-1H-benzimidazole (EZ07B)

Purification of the raw product (79 % yield) by column chromatography (silica gel, eluent: ethyl acetate gave the pure product in 64 % yield as yellow orange crystals. (Substance characterization see above.)

**1-*tert*.-Butoxycarbonyl-2-[(4-nitrophenoxy)-methyl]-1*H*-benzimidazole** (EZ13A) Purification of the raw product (89 % yield) by column chromatography (silica gel, eluent: ethyl acetate gave the pure product in 40 % yield as light yellow crystals, mp 149-151° C. ¹H-NMR (DMSO-d₆) δ [ppm]: 1.61 (s, 9H, (CH₃)₃), 5.69 (s, 2H, CH₂), 7.25-7.46 (m, 4H, arene H), 7.67-7.71 (m, 1H, arene H), 7.97 (d, J = 8.0 Hz, 1H, arene H), 8.16 - 8.26 (m, 2H, arene H). IR [cm⁻¹]: 1750 (C=O)

### Step b)

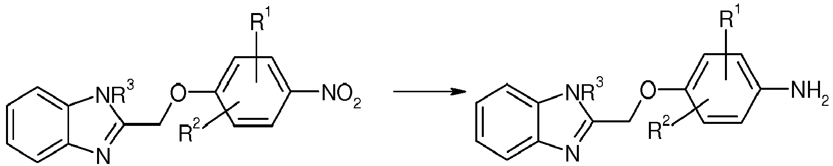

A mixture of 1 equivalent of the appropriate nitro compound, 5 equivalents of ammonium formate and Pd/C (10 %) (ca. 0.1 g per mmol) in methanol (ca. 25 mL per mmol) was stirred under an argon atmosphere at 48 °C until the starting material was consumed as determined by tlc (ca. 1-3 h). Then, the catalyst was filtered off, the solvent was removed *in vacuo,* and the residue was taken up in dichloromethane. This solution was washed with water and brine, dried over anhydrous sodium sulphate and evaporated.

### 2-[(4-Amino-3-methylphenoxy)-methyl]-1-tert.-butoxycarbonyl-1H-benzimidazole (EZ09A)

The raw product (91 % yield) was purified by column chromatography (silica gel, eluent: ethyl acetate/dichloromethane (2/1) and subsequent recrystallization from isopropanol to give the pure compound as light pink crystals in 84 % yield, mp 117-118° C. ¹H-NMR (DMSO-d₆) δ [ppm]: 1.58 (s, 9H, (CH₃)₃), 2.02 (s, 3H, CH₃), 4.42 (s, 2H, D₂O exchangeable, NH₂), 5.31 (s, 2H, CH₂), 6.50-6.66 (m, 3H, arene H), 7.31-7.45 (m, 2H, arene H), 7.69-7.73 (m, 1H, arene H), 7.94-7.98 (m, 1H, arene H). IR [cm⁻¹]: 1737 (C=O), 3226, 3314 (NH)

### 2-[(4-Aminophenoxy)-methyl]-1-tert.-butoxycarbonyl-1H-benzimidazole (EZ16C)

The raw product (76 % yield) was purified by column chromatography (silica gel, eluent: ethyl acetate/dichloromethane (2/1) to give the pure compound as light pink crystals in 54 % yield, mp 100-101° C. ¹H-NMR (DMSO-d₆) δ [ppm]: 1.58 (s, 9H, (CH₃)₃), 4.65 (s, 2H, D₂O exchangeable, NH₂), 5.31 (s, 2H, CH₂), 6.50 (d, J = 8.8 Hz, 2H, arene H), 6.73 (d, J = 8.8 Hz, 2H, arene H), 7.31-7.44 (m, 2H, arene H), 7.69-7.73 (m, 1H, arene H), 7.94-7.98 (m, 1H, arene H). IR [cm⁻¹]: 1741 (C=O), 3221, 3342 (NH).

### Step c) to obtain a urea compound

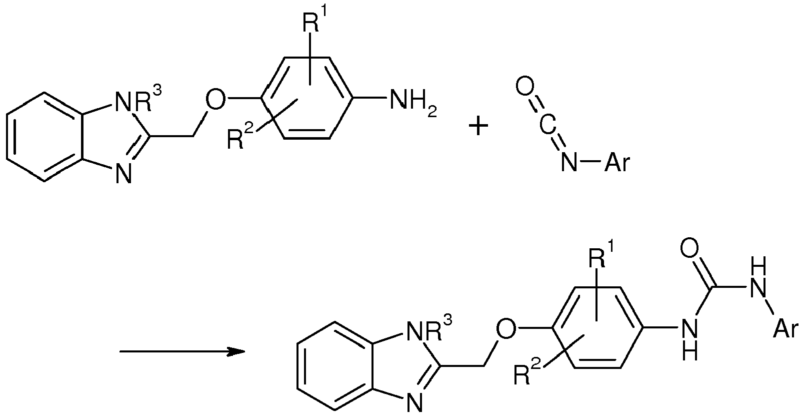

A solution of 1.1 equivalents of 3,4-dichlorophenylisocyanate in dry THF (ca. 2 mL per mmol) was added dropwise to a mixture of 1 equivalent of the appropriate amino derivative in dry THF (ca. 2 mL per mmol) and the resulting reaction mixture was stirred at room temperature until the starting material was consumed as determined by tlc (ca. 1-2 h). Then, the precipitate was isolated by vacuum filtration, washed with THF and diethyl ether and recrystallized from a suitable solvent to yield the pure product.

### N-[4-(1-tert.-Butoxycarbonyl-1H-benzimidazol-2-ylmethoxy)-2-methylphenyl]-N'-(3,4-dichlorophenyl)-urea (EZ12)

Purification of the raw product (69 % yield) by recrystallization from ethanol gave the pure product in 13 % yield as a white powder, mp 261-263 °C. ¹H-NMR (DMSO-d₆) δ [ppm]: 1.61 (s, 9H, (CH₃)₃), 2.19 (s, 3H, CH₃), 5.45 (s, 2H, CH₂), 6.81-6.91 (m, 2H, arene H), 7.25-7.51 (m, 5H, arene H), 7.69-7.74 (m, 1H, arene H), 7.88-7.98 (m, 3H, arene H, NH; 2H after D₂O-exchange), 9.15 (s, 1H, D₂O exchangeable, NH). IR [cm⁻¹]: 1702, 1742 (C=O), 3263, 3356 (NH). Calcd. for C₂₇H₂₆Cl₂N₄O₄ x 0.8 H₂O (555.85): C, 58.34; H, 5.00; N, 10.00. Found C, 58.33; H, 4.98; N, 9.97.

### N-[4-(1-tert.-Butoxycarbonyl-1H-benzimidazol-2-ylmethoxy)-phenyl]-N'-(3,4-dichlorophenyl)-urea (EZ18B)

Purification of the raw product (79 % yield) by recrystallization from ethyl acetate gave the pure product in 61 % yield as a white powder, mp 262-264 °C. ¹H-NMR (DMSO-d₆) δ [ppm]: 1.59 (s, 3H, (CH₃)₃), 5.45 (s, 2H, CH₂), 6.98 (d, J = 9.2 Hz, 2H, arene H), 7.27-7.51 (m, 6H, arene H), 7.69-7.73 (m, 1H, arene H), 7.86 (d, J = 2.4 Hz, 1H, arene H), 7.95-8.00 (m, 1H, arene H), 8.63 (s, 1H, D₂O exchangeable, NH), 8.92 (s, 1H, D₂O exchangeable, NH). IR [cm⁻¹]: 1749, 1708 (C=O). Calcd. for C₂₆H₂₄Cl₂N₄O₄ (527.41): C, 59.21; H, 4.59; N, 10.62. Found: C, 59.08; H, 4.64; N, 10.38.

### Step c) to obtain a thiourea compound

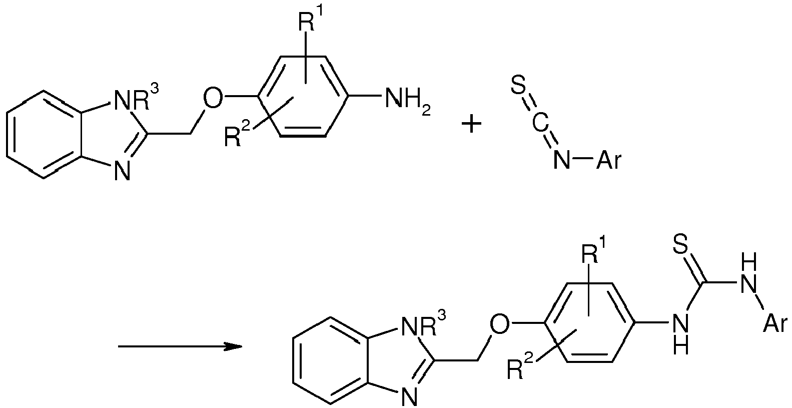

A solution of 1.1 equivalents of 3,4-dichlorophenylisothiocyanate in dry THF (ca. 2 mL per mmol) was added under ice cooling dropwise to a mixture of 1 equivalent of the appropriate amino derivative in dry THF (ca. 2-10 mL per mmol) and the resulting reaction mixture was stirred at room temperature until the starting material was consumed as determined by tlc (ca. 1-2 h). Then, the solvent was removed *in-vacuo* and the residue was purified as described below.

### N-[4-(1-tert.-Butoxycarbonyl-1H-benzimidazol-2-ylmethoxy)-2-methylphenyl]-N'-(3,4-dichlorophenyl)-thiourea (EZ15)

Purification of the raw product (100 % yield) by column chromatography (silica gel, eluent: dichloromethane/ethyl acetate (5/1) and subsequent recrystallization from ethanol/tetrahydrofuran (ca. 1/1) gave the pure product in 76 % yield as a white powder, mp 172 °C. ¹H-NMR (DMSO-d₆) δ [ppm]: 1.62 (s, 9H, (CH₃)₃), 2.19 (s, 3H, CH₃), 5.49 (s, 2H, CH₂), 6.84-6.96 (m, 2H, arene H), 7.12 (d, J = 8.6 Hz, 1H, arene H), 7.31-7.46 (m, 3H, arene H), 7.54 (d, J = 8.8 Hz, 1H, arene H), 7.70-7.74 (m, 1H, arene H), 7.89 (d, J = 2.2 Hz, 1H, arene H), 7.95-7.99 (m, 1H, arene H), 9.47 (s (br), 1H, D₂O exchangeable, NH), 9.72 (s (br), 1H, D₂O exchangeable, NH). IR [cm⁻¹]: 1749 (C=O), 3180, 3350 (NH). Calcd. for: C₂₇H₂₆Cl₂N₄O₃S (557.50): C, 58.17; H, 4.70; N, 10.05; S, 5.75. Found: C, 58.19; H, 4.68; N, 10.00; S, 5.71.

### N-[4-(1-tert.-Butoxycarbonyl-1H-benzimidazol-2-ylmethoxy)-phenyl]-N'-(3,4-dichlorophenyl)-thiourea (EZ20)

Purification of the raw product by column chromatography (silica gel, eluent: dichloromethane/ethyl acetate (5/1) and subsequent recrystallization from ethyl acetate gave the pure product in 61 % yield, as a white solid, mp 165-167 °C. ¹H-NMR (DMSO-d₆) δ [ppm]: 1.61 (s, 9H, (CH₃)₃), 5.50 (s, 2H, CH₂), 7.02 (d, J = 8.8 Hz, 2H, arene H), 7.30-7.45 (m, 5H, arene H), 7.55 (d, J = 8.8 Hz, 1H, arene H), 7.70-7.74 (m, 1H, arene H), 7.87 (d, J = 2.0 Hz, 1H, arene H), 7.95-7.99 (m, 1H, arene H), 9.85 (s, 2H, D₂O exchangeable, 2x NH). IR [cm⁻¹]: 1751 (C=O), 3159, 3363 (NH). Calcd. for C₂₆H₂₄Cl₂N₄O₃S (543.47): C, 57.46; H, 4.45; N, 10.31; S, 5.90. Found: C, 57.45; H, 4.45; N, 10.26; S, 5.86.

### Step d)

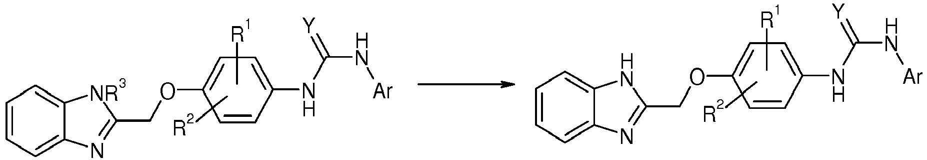

Ten equivalents of trifluoroacetic acid were added at 0-20 °C to a suspension of 1 equivalent of the appropriate *N*-*tert*.-butoxycarbonyl protected derivative in dichloromethane (30-40 mL/mmol). The resulting reaction mixture was stirred at 30 °C until the starting material was consumed as determined by tlc (ca. 5 h). Then, saturated sodium bicarbonate solution was added at 0 °C, the solution was extracted with methylene chloride and the organic layer was washed with water and evaporated *in vacuo* to dryness. The residue was treated with water, the reaction product was isolated by vacuum filtration, washed with water and dried to constant weight.

### N-[4-(1H-Benzimidazol-2-ylmethoxy)-2-methylphenyl]-N'-(3,4-dichlorophenyl)-urea (EZ14)

Purification of the raw product (94 % yield) by recrystallization from 1,4-dioxane gave the pure product in 35% yield as white powder, mp 245-246° C. ¹H-NMR (DMSO-d₆) δ [ppm]: 2.21 (s, 3H, CH₃), 5.39 (s, 2H, CH₂), 6.89-6.96 (m, 2H, arene H), 7.27-7.33 (m, 3H, arene H), 7.47-7.54 (m, 2H, arene H), 7.62-7.67 (m, 2H, arene H), 7.89 (d, 1H, J = 2.0 Hz, arene H), 8.05 (s, 1H, D₂O exchangeable, NH), 9.27 (s, 1H, D₂O exchangeable, NH). IR [cm⁻¹]: 1639 (C=O), 3278 (NH). Calcd. for C₂₂H₁₈Cl₂N₄O₂ x 0.6 H₂O (452.13): C, 58.44: H, 4.28; N, 12.39. Found: C, 58.57; H, 4.07; N, 12.06.

### N-[4-(1-H-Benzimidazol-2-ylmethoxy)-phenyl]-N'-(3,4-dichlorophenyl)-urea (EZ19)

Purification of the raw product (100 % yield) by column chromatography (silica gel, eluent: dichloromethane/tetrahydrofuran (7/1) to pure tetrahydrofuran) and subsequent recrystallization from isopropanol gave the pure product in 31% yield as white powder, mp 272° C. ¹H-NMR (DMSO-d₆) δ [ppm]: 5.27 (s, 2H, CH₂), 7.02 (d, J = 8.8 Hz, 2H, arene H), 7.11-7.38 (m, 5H, arene H), 7.47-7.62 (m, 3H, arene H), 7.86 (d, J = 2.6 Hz, 1H, arene H), 8.63 (s, 1H, D₂O exchangeable, NH), 8.91 (s, 1H, D₂O exchangeable, NH), 12.65 (s, 1H, D₂O exchangeable, NH). IR [cm⁻¹]: 1636 (C=O), 3277 (NH). Calcd. for C₂₁H₁₆Cl₂N₄O₂ x 0.2 H₂O (430.90): C, 58.54; H, 3.84; N, 13.00. Found: C, 58.64; H, 3.72; N, 12.56.

### N-[4-(1H-Benzimidazol-2ylmethoxy)-2-methylphenyl]-N'-(3,4-dichlorophenyl)-thiourea (EZ17)

Purification of the raw product (81 % yield) by column chromatography (silica gel, eluent: dichloromethane/tetrahydrofuran (7/2) to pure tetrahydrofuran) and subsequent recrystallization-from methanol gave the pure product in 52 % yield as colourless powder, mp 123-125° C. ¹H-NMR (DMSO-d₆) δ [ppm]: 2.19 (s, 3H, CH₃), 5.30 (s, 2H, CH₂), 6.89-7.00 (m, 2H, arene H), 7.11-7.21 (m, 3H, arene H), 7.40-7.63 (m, 4H, arene H), 7.88 (d, J = 2.2 Hz, 1H, arene H), 9.47 (s (br), 1H, D₂O exchangeable, NH), 9.71 (s (br), 1H, D₂O exchangeable, NH), 12.67 (s (br), 1H, D₂O exchangeable, NH). IR [cm⁻¹]: no characteristic absorption bands. Calcd. for C₂₂H₁₈Cl₂N₄OS (457.40): C, 57.77; H, 3.97; N, 12.25; S, 7.01. Found: C, 57.63; H, 3.87; N, 12.04; S, 6.79.

### N-[4-(1-H-Benzimidazol-2-ylmethoxy)-phenyl]-N'-(3,4-dichlorophenyl)-thiourea (EZ21)

Purification of the raw product (71 % yield) by column chromatography (silica gel, eluent: dichloromethane/tetrahydrofuran (7/1) to pure tetrahydrofuran) and subsequent treatment with diisopropyl ether/dichloromethane (1/1) gave the pure product in 43 % yield as white powder, mp 160-162 °C. ¹H-NMR (DMSO-d₆) δ [ppm]: 5.31 (s, 2H, CH₂), 7.05-7.24 (m, 4H, arene H), 7.29-7.63 (m, 6H, arene H), 7.86 (d, J = 2.2 Hz, 1H, arene H), 9.83 (s, 1H, D₂O exchangeable, NH), 9.85 (s, 1H, D₂O exchangeable, NH) 12.68 (s, 1H, D₂O exchangeable, NH). IR [cm⁻¹]: 3190, 3203 (NH). Calcd. for C₂₁H₁₆Cl₂N₄OS (443.36): C, 56.89; H, 3.64; N, 12.64; S, 7.23. Found: C, 56.96, H, 3.61, N, 12.30; S, 7.05.

### Example 9: Synthesis of benzoxazole compounds according to formula II

As both, the benzthioazole compound (I-12, X is S) and the analogous benzimidazole compound (EZ14, X is NH), showed good activity. The inventors also synthesized three compounds with benzoxazole moiety (X is O) with urea (SE-04) and thiourea motive (SE-12, TE-12). The characteristic steps a) to c) according to the invention were maintained, while the individual conditions were varied in comparison to the synthesis of benzimidazole compounds.

### Step a)

### Synthesis of 2-(4-nitrophenoxy)benzoxazoles

2-(3-Methyl-4-nitrophenoxy)benzoxazole (CAS Registry Number 927538-21-4) and 2-(4-nitrophenoxy)benzoxazole (CAS Registry Number 405900-54-1) are already known. These compounds as well as substituted derivatives can be prepared starting from the appropriate 2-chloromethylbenzoxazole and unsubstituted or substituted 4-nitrophenol in an inert solvent in the presence of base.

### Step b)

### Synthesis of 2-(4-aminophenoxy)benzoxazoles

The amino compounds are available by reduction of the corresponding nitro substituted derivatives: A mixture of the appropriate 2-(4-nitrophenoxy)benzoxazole in methanol was reduced on a Parr hydrogenator at 40 psi using Raney nickel catalyst until the reduction was complete (determined by tlc). Then the mixture was filtered to remove the catalyst and the filtrate was evaporated under reduced pressure to yield the pure compound in nearly quantitative yield.

### 2-(4-Amino-3-methylphenoxy)benzoxazole (SE-03)

IR [cm⁻¹]: 3425, 3335 (NH₂). ¹H-NMR (DMSO-d₆) δ [ppm]: 2.01 (s, 3H, CH₃), 4,46 (s, 2H, D₂O exchangeable, NH₂), 5.25 (s, 2H, CH₂), 6.52 (d, J= 8.6 Hz, 1H, arene H), 6.65 (dd, J = 8.6 Hz, J = 3.0 Hz, 1H, arene H), 6.71 (d, J = 3.0 Hz, 1H, arene H), 7.33-7.46 (m, 2H, arene H), 7.70-7.80 (m, 2H, arene H). Calcd for C₁₅H₁₄N₂O₂ x 0,1 H₂O (256.09): C, 70.35; H, 5.59; N, 10.94. Found: C, 70.40; H, 5.53; N, 10.99.

### Step c)

To a solution of 1.0 equivalent of the appropriate 2-(4-aminophenoxy)benzoxazole in dry tetrahydrofuran (ca. 3 mL/mmol) was added dropwise a solution of 1.1 equivalents of the appropriate isocyanate or isothiocyanate in dry tetrahydrofuran (ca. 1 mL/mmol) and the reaction mixture was stirred at room temperature until the starting material was completely consumed (tlc monitoring). Then the solid obtained was collected by filtration, washed with tetrahydrofuran, and dried. The product was further purified by treatment with acetone or by recrystallization from a suitable solvent.

### N-[4-(2-Benzoxazolylmethoxy)-2-methylphenyl]-N'-(3,4-dichlorophenyl)-urea (SE-04)

Yield: 79 % of colourless crystals, IR [cm⁻¹]: 3280 (br, NH), 1636 (C=O). ¹H-NMR (DMSO-d₆) δ [ppm]: 2.19 (s, 3H, CH₃), 5.42 (s, 2H, CH₂), 6.87-6.95 (m, 2H, arene H), 7.25-7.30 (m, 1H, arene H), 7.35-7.53 (m, 4H, arene H), 7.73-7.79 (m, 2H, arene H), 7.88 (d, J = 2.2 Hz, 1H, arene H), 7.93 (s, 1H, D₂O exchangeable, NH), 9.15 (s, 1H, D₂O exchangeable, NH). Calcd for C₂₂H₁₇Cl₂N₃O₃ (442.30): C, 59.74; H, 3.87; N, 9.50. Found: C, 59.73; H, 3.86; N, 9.40.

### N-[4-(Benzoxazol-2-ylmethoxy)-2-methyl-phenyl]-N'-phenylthiourea (SE-12)

Yield: 72 % of beige crystals, IR [cm⁻¹]: 3151 (NH). ¹H-NMR (DMSO-d₆) δ [ppm]: 2.20 (s, 3H, CH₃), 5.45 (s, 2H, CH₂), 6.87-6.99 (m, 2H, arene H), 7.06-7.16 (m, 2H, arene H), 7.26-7.35 (m, 2H, arene H), 7.38-7.48 (m, 4H, arene H), 7.72-7.81 (m, 2H, arene H), 9.20 (s,1H, D₂O exchangeable, NH), 9.56 (s, 1H, D₂O exchangeable, NH). Calcd for C₂₂H₁₉N₃O₂S; (389.47): C, 67.84; H, 4.92; N, 10.79; S, 8.23. Found: C, 67.87; H, 4.94; N, 10.84; S, 8.07.

### N-[4-(Benzoxazol-2-yl)-methoxyphenyl]-N'-phenylthiourea (TS-12)

Yield: 88 % of colourless crystals, IR [cm⁻¹]: 3166, 3185 (NH). ¹H-NMR (DMSO-d₆) δ [ppm]: 5.45 (s, 2H, CH₂), 7.03-7.13 (m, 3H, arene H), 7.26-7.47 (m, 8H, arene H), 7.72-7.80 (m, 2H, arene H), 9.63 (s, 2H, D₂O exchangeable, 2 x NH). Calcd for C₂₁H₁₇N₃O₂S (375.45): C, 67.18; H, 4.56; N, 11.19. Found: C, 67.00; H, 4.65; N, 11.03.

### Example 10: Synthesis of a guanidine compounds according to formula II

Guanidine derivatives are available by reaction of a carbodiimide with an amine (step f). The carbodiimides can be prepared from an appropriate thiourea or a derivative thereof by different methods, for example by reaction of a *N,N'*-disubstituted thiourea with methanesulfonyl chloride in the presence of base or *via* a *S*-substituted isothiourea with silver nitrate (step e).

### N-[4-(Benzoxazol-2-ylmethoxy)-2-methylphenyl]-N'-phenylcarbodiimide (SE-26a)

A solution of 2.0 equiv. of methanesulfonyl chloride in dichloromethane (ca. 1 mL/mmol) was added dropwise to an ice-cooled suspension of 1.0 equiv. of *N*-[4-(benzoxazol-2-ylmethoxy)-2-methyl-phenyl]-*N'*-phenylthiourea, 3.0 equiv. of triethylamine and catalytic amounts of 4-(*N,N*-dimethylamino)pyridine in dichloromethane (ca. 25 mL/mmol thiourea). The reaction mixture was stirred at 0 °C until the starting material was completely consumed (tlc monitoring). Then the dichloromethane solution was washed with water and brine, dried over anhydrous sodium sulfate, evaporated and purified by column chromatography (silica gel, eluent: ethyl acetate/petroleum ether (4/1) to give the product in quantitative yield. IR [cm⁻¹]: 2107 (N=C=N). ¹H-NMR (DMSO-d₆) δ [ppm]: 2.28 (s, 3H, CH₃), 5.44 (s, 2H, CH₂), 6.90-7.03 (m, 2H, arene H), 7.15-7.21 (m, 4H, arene H), 7.33-7.46 (m, 4H, arene H), 7.72-7.79 (m, 2H, arene H).

### N-[4-(Benzoxazol-2-ylmethoxy)-2-methyl-phenyl]-N'-cyclopropyl-N"-phenyl-guanidine (SE-41a)

A mixture of 1.0 equiv. of *N*-[4-(benzoxazol-2-ylmethoxy)-2-methylphenyl]-*N'*-phenyl-carbodiimide and 1.75 equiv. of cyclopropylamine in dichloromethane (ca. 25 mL/mmol carbodiimide) was stirred at room temperature until the starting material was completely consumed (tlc monitoring). The solvent was removed and the raw product was purified by column chromatography (silica gel, eluent: ethyl acetate/petroleum ether (4/1) to yield 34 % of the pure compound. IR [cm⁻¹]: 3395 (NH-st), 1636 (C=N). ¹H-NMR (DMSO-d₆) δ [ppm]: 0.36-0.57 (m, 4H, 2xCH₂), 2.09 (s, 3H, CH₃), 2.36-2.48 (m, 1H, CH), 5.33 (m, 2H, CH₂), 5.42 (s br, 1H, D₂O exchangeable, NH), 6.75-6.85 (m, 4H, arene H), 7.10-7.18 (m, 3H, arene H), 7.34-7.47 (m, 3H, 2H after D₂O exchange, NH, arene H), 7.71-7.79 (m, 2H, arene H). Calcd. for C₂₅H₂₄N₄O₂, x 0.1 H₂O x 0.3 ethyl acetate (426,71): C, 71.50; H, 6.00; N, 13.13. Found: C, 71.37; H, 6.01; N, 12.81.

### N-[4-(Benzoxazol-2-yl)-methoxyphenyl]-S-methyl-N'-phenyl-isothiourea (TS-16b)

A solution of 1.5 equiv. of methyliodide in acetone was added dropwise to an ice-cooled mixture of 1.0 equiv. of *N*-[4-(benzoxazol-2-yl)-methoxyphenyl]-*N'*-phenylthiourea and 1.0 equiv. of potassium carbonate in acetone (ca 5 mL/mmol). After stirring for 2 h at 0 °C, the mixture was warmed to room temperature and stirred at this temperature until the starting material was completely consumed (tlc monitoring). Then it was filtered and evaporated. The residue was taken up in ethyl acetate, washed with water and brine, dried over anhydrous sodium sulfate and the solvent was removed *in vacuo* to yield 81 % of the raw product which can be directly used for further derivatization.

### N-[4-(Benzoxazol-2-yl)-methoxyphenyl]-N'-phenylcarbodiimide (TS-21b)

To a solution of 1.0 equiv. of *N*-[4-(benzoxazol-2-yl)-methoxyphenyl]-*S*-methyl-*N'*-phenyl-isothiourea in acetonitrile (ca. 2 mL/mmol) were successively added dropwise a solution of 1.0 equiv. of silver nitrate in acetonitrile and 1.0 equiv. of trimethylamine and the reaction mixture was stirred at room temperature until the starting material was completely consumed (tlc monitoring, ca. 5 min.). The precipitate thus obtained was removed by filtration, washed with acetonitrile and the filtrate was evaporated. The residue was taken up in ethyl acetate, washed with water and brine, dried over anhydrous sodium sulfate and the solvent was removed *in vacuo* to yield 91 % of the raw product which can be directly used for further derivatization. IR [cm⁻¹]: 2103 (N=C=N).

### N-[4-(Benzoxazol-2-yl)-methoxyphenyl]-N'N'-diethyl-N"-phenylguanidine (TS-27b)

A mixture of 1.0 equiv. of *N*-[4-(Benzoxazol-2-yl)-methoxyphenyl]-*N'*-phenylcarbodiimide and 3.0 equiv. of diethylamine in dichloromethane (ca. 50 mL/mmol carbodiimide) was stirred at room temperature until the starting material was completely consumed (tlc monitoring). The solvent was removed, the residue was taken up in ethyl acetate, washed with water and brine, dried over anhydrous sodium sulfate and the solvent was removed *in vacuo.* The raw product was purified by column chromatography (silica gel, eluent: tetrahydrofuran) followed by recrystallization from ethyl acetate to yield 45 % of the pure guanidine as ochery crystals. IR [cm⁻¹]: 3308, 3357 (NH), 1674 (C=N). ¹H-NMR (DMSO-d₆) δ [ppm]: 1.06 (t, J = 7.0 Hz, 6H, 2 x CH₃), 3.27 (q, J = 7.0 Hz, 4H, 2x CH₂), 4.55 (s, 2H, CH₂), 6.68-7.09 (m, 12H, arene H), 8.00 (dd, J = 7.8 Hz, J = 1.4 Hz, 1H, arene H), 9.07 (s br, 1H, D₂O exchangeable, NH). Calcd. for C₂₅H₂₆N₄O₂, x 0.9 H₂O x 0.2 ethyl acetate (448.35): C, 69.12; H, 6.61; N, 12.50. Found: C, 69.06; H, 6.54; N, 12.55.

### Example 11: Potential synthesis routes for further compounds according to formula I

Synthesis of compound with ethenylen (L is CH=CH) or ethylen (L is CH₂-CH₂) spacer should be performed similar to the disclosed reaction in regard to step c) i.e. by coupling of an amine with an isocyanate (Ar-N=C=O) or isothiocyanate (Ar-N=C=S). In order to obtain the respective amine alternative steps have to be performed. Fortunately, synthesis of similar amines has been described before (Herner, A.; Nikic, I.; Kállay, M.; Lemke, E.A.; Kele, P.; Org. Biol. Chem., 2013, 11, 3297-3306). The starting nitro-compounds are known 2-(4-nitrostryryl)-1,3-benzothiazole (CAS 84645-69-2, X=S) or 2-(4-nitrostryryl)-benzoxazole (CAS 3271-27-0, X=O). Depending on the reduction conditions these compounds may be reduced to amine compounds with ethenylen linker (CAS 101645-25-4, wherein X is S; CAS 101645-24-3, wherein X is O) as described by Herner et al. Alternatively reduction conditions are described in DE 31 48 291 A1 and also reduce the linker resulting in CAS 86912-94-9 (X is S) or CAS 39921-38-5 (X is O).

Compounds with a thioether linker (wherein L is CH₂-S) should be available using the synthetic strategy as described for the ether linker (L is CH₂-O, compound according to formula II). The only difference would be the usage of 4-nitro-thiophenol instead of 4-nitrophenol in step a) of the process for synthesizing compounds according to formula II. Individual intermediate products are known (CAS: 100537-48-2 is a nitro compound wherein X is S, or CAS 1095513-65-7 is an amine compound wherein X is O).

## Claims

1. A compound according to formula I 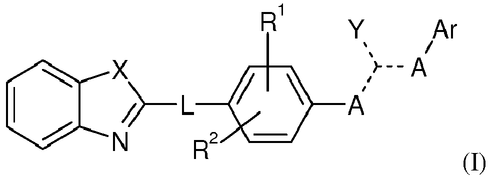
wherein L is CH₂-O, CH₂-S, CH₂-CH₂, or CH=CH;
R¹ and R² are independently selected out of H, halogen, C1 to C3 alkyl, halogenated C1 to C3 alkyl, C1 to C3 alkyloxy, or halogenated C1 to C3 alkyloxy;
Ar is an aryl moiety, preferably phenyl or naphthyl, or a moncyclic- or bicyclic heteroaryl moiety, wherein optionally the aryl or heteroaryl moiety is further substituted;
X is O, S, or NR³, wherein R³ is H, C1 to C3 alkyl, halogenated C1 to C3 alkyl, or *tert*-butyloxycarbonyl;
Y is O, S, NR⁴ or N(R⁴)R⁵ wherein R⁴ and R⁵ are independently selected out of H or C1 to C3 alkyl; and
wherein if Y is O, S, or NR⁴, then both A are NH, and the punctuated bonds represent a double bond to Y and single bonds to each A; or
wherein if Y is N(R⁴)R⁵, then one A is N, another A is NH, and the punctuated bonds represent a double bond to the A being N, a single bond to the A being NH and a single bond to Y;
with the provision that a compound in which L is CH₂-O, X is NH, Y is S, both A are NH and both R¹ and R² are H is excluded,
for use as a medicament.

2. A compound a compound according to formula I 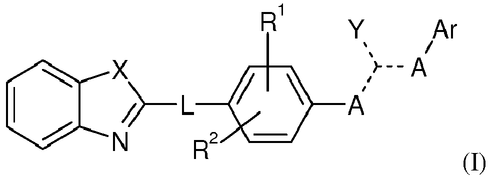
wherein L is CH₂-O, CH₂-S, CH₂-CH₂, or CH=CH;
R¹ and R² are independently selected out of H, halogen, C1 to C3 alkyl, halogenated C1 to C3 alkyl, C1 to C3 alkyloxy, or halogenated C1 to C3 alkyloxy;
Ar is an aryl moiety, preferably phenyl or naphthyl, or a moncyclic- or bicyclic heteroaryl moiety, wherein optionally the aryl or heteroaryl moiety is further substituted;
X is O, S, or NR³, wherein R³ is H, C1 to C3 alkyl, halogenated C1 to C3 alkyl, or *tert*-butyloxycarbonyl;
Y is O, S, NR⁴ or N(R⁴)R⁵ wherein R⁴ and R⁵ are independently selected out of H or C1 to C3 alkyl; and
wherein if Y is O, S, or NR⁴, then both A are NH, and the punctuated bonds represent a double bond to Y and single bonds to each A;
wherein if Y is N(R⁴)R⁵, then one A is N, another A is NH, and the punctuated bonds represent a double bond to the A being N, a single bond to the A being NH and a single bond to Y"
for use in treatment or prevention of an inflammatory disease or an inflammation-related disorder.

3. A compound for use according to claim 2, for treatment or prevention of an inflammatory disease, wherein preferably the inflammatory disease is selected out of the group consisting of rheumatoid arthritis, asthma, allergic rhinitis, atherosclerosis, Alzheimer's disease or Parkinson's disease.

4. A compound for use according to any of claims 1 to 3, wherein L is CH₂-O or CH₂-CH₂, preferably CH₂-O.

5. A compound for use according to any of claims 1 to 4, wherein X is S or NR³, wherein preferably NR³ is NH.

6. A compound according to formula II 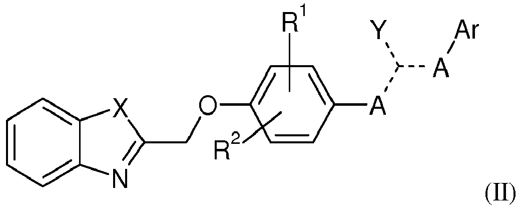
wherein R¹ and R² are independently selected out of H, halogen, C1 to C3 alkyl, halogenated C1 to C3 alkyl, C1 to C3 alkyloxy, or halogenated C1 to C3 alkyloxy;
Ar is an aryl moiety, preferably phenyl or naphthyl, or a moncyclic- or bicyclic heteroaryl moiety, wherein optionally the aryl or heteroaryl moiety is further substituted;
X is O or NR³, wherein R³ is H, C1 to C3 alkyl, halogenated C1 to C3 alkyl, or tert-butyloxycarbonyl;
Y is O, S or NR⁴ or N(R⁴)R⁵ wherein R⁴ and R⁵ are independently selected out of H or C1 to C3 alkyl; and
wherein if Y is O, S, or NR⁴, then both A are NH, and the punctuated bonds represent a double bond to Y and single bonds to each A; or
wherein if Y is N(R⁴)R⁵, then one A is N, the other A is NH, and the punctuated bonds represent a double bond to the A being N, a single bond to the A being NH and a single bond to Y;
with the provision that a compound in which L is CH₂-O, X is NH, Y is S, both A are NH and both R¹ and R² are H is excluded.

7. A compound according to claim 6, wherein X is NR³ and wherein R³ is H, C1 to C3 alkyl, halogenated C1 to C3 alkyl, or *tert*-butyloxycarbonyl, preferably R³ is H or *tert*-butyloxycarbonyl.

8. A compound for use according to any of claims 1 to 5 or a compound according to any of claims 6 to 7, wherein Ar is a phenyl group, optionally the phenyl substituted with one or more of the moieties selected out of the group comprising F, Cl or CF₃.

9. A compound for use according to any of claims 1 to 5 or 8 or a compound according to any one of claims 6 to 8, wherein at least one of R¹ or R² is not H.

10. A compound for use according to any of claims 1 to 5 or 8 to 9 according to formula III 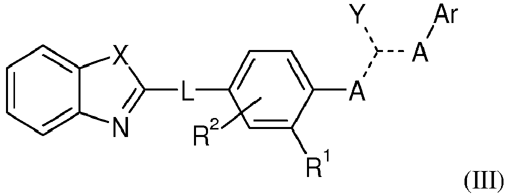 or a compound according any one of claims 6 to 9 according to formula IV 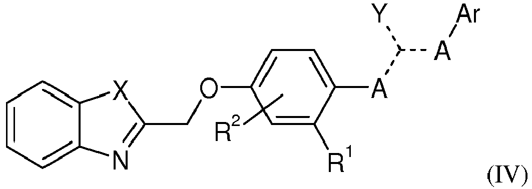
wherein R¹ is selected out of halogen, C1 to C3 alkyl, halogenated C1 to C3 alkyl, C1 to C3 alkyloxy, or halogenated C1 to C3 alkyloxy, preferably C1 to C3 alkyl, more preferably C1 alkyl; and
wherein R² is selected out of H, halogen, C1 to C3 alkyl, halogenated C1 to C3 alkyl, C1 to C3 alkyloxy, or halogenated C1 to C3 alkyloxy, preferably H.

11. A compound for use according to any of claims 1 to 5 or 8 to 10 according to formula V 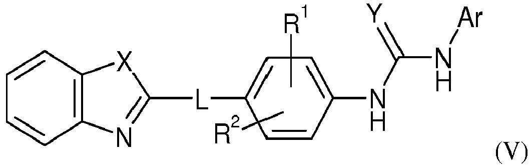 or a compound according any one of claims 6 to 10 according to formula VI 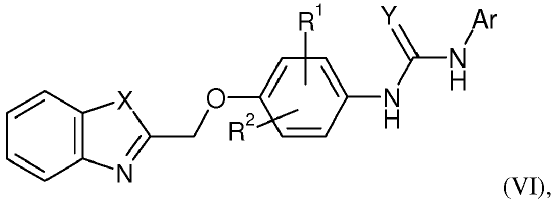 wherein Y is O or S, preferably O.

12. A compound for use according to any one of claims 1 to 5 or 8 to 11 or a compound according to any one of claims 6 to 11, wherein the compound is capable of inhibiting biochemically evaluated activity of soluble EH with concentrations of 0.1 µM or lower and 5-LOX product formation with concentrations of 1 µM or lower.

13. A pharmaceutical composition comprising a compound for use according to any one of claims 1 to 5 or 8 to 12 or a compound according to any one of claims 6 to 12 in association with at least one pharmaceutical excipient.

14. Process for synthesis of a compound according to formula II 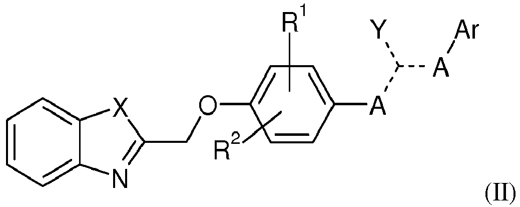
wherein R¹ and R² are independently selected out of H, halogen, C1 to C3 alkyl, halogenated C1 to C3 alkyl, C1 to C3 alkyloxy, or halogenated C1 to C3 alkyloxy;
Ar is an aryl moiety, preferably phenyl or naphthyl, or a moncyclic- or bicyclic heteroaryl moiety, wherein optionally the aryl or heteroaryl moiety is further substituted;
X is O, S, or NR³, wherein R³ is H, C1 to C3 alkyl, halogenated C1 to C3 alkyl, or *tert*-butyloxycarbonyl;
Y is O, S, NR⁴ or N(R⁴)R⁵, wherein R⁴ and R⁵ are independently selected out of H or C1 to C3 alkyl; and
wherein if Y is O, S, or NR⁴, then both A are NH, and the punctuated bonds represent a double bond to Y and single bonds to each A; or
wherein if Y is N(R⁴)R⁵, then one A is N, the other A is NH, and the punctuated bonds represent a double bond to the A being N, a single bond to the A being NH and a single bond to Y;
comprising the steps of
a) providing a mixture of a compound according to formula VII 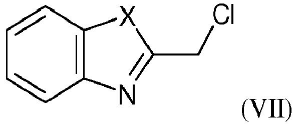 and a compound according to formula VIII 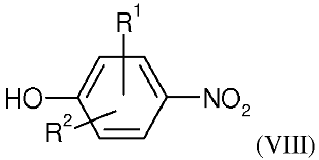 in conditions that allow them to form an ether and obtain the ether according to formula IX 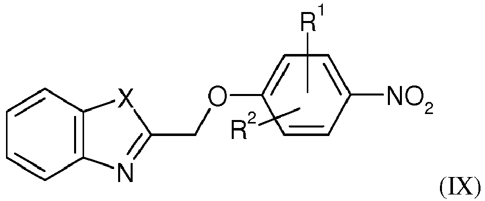
wherein X is O, S, or NR³, wherein R³ is H, C1 to C3 alkyl, halogenated C1 to C3 alkyl, or *tert*-butyloxycarbonyl,
R¹ and R² are independently selected out of H, halogen, C1 to C3 alkyl, halogenated C1 to C3 alkyl, C1 to C3 alkyloxy, or halogenated C1 to C3 alkyloxy,
b) treating the compound according to formula IX obtained in step b) with molecular hydrogen or a source for molecular hydrogen and a metal catalyst under conditions that allow reduction of the compound according to formula IX to obtain an amino compound according to formula X 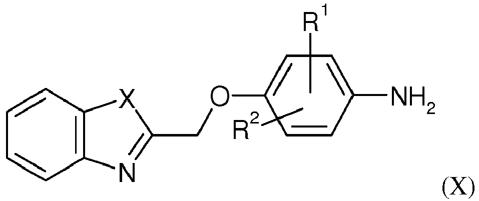 wherein X, R¹ and R² are defined as above
c) treating the amino compound according to formula X obtained in step b) with a compound according to formula XI or formula XII 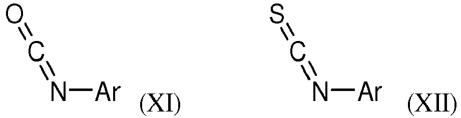 under conditions that a allow formation of an compound according to formula VI 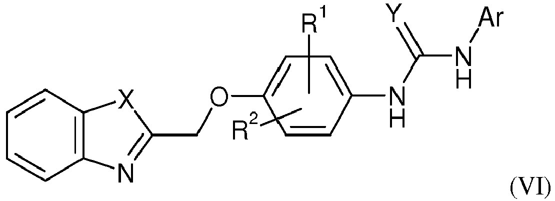
wherein Y is O, when the amino compound is treated with a compound according to formula XI and Y is S, when the amino compound is treated with a compound according to formula XII,
Ar is an aryl moiety, preferably a phenyl or naphthyl moiety, or a moncyclic- or bicyclic heteroaryl group, wherein the aryl or heteroaryl group is optionally further substituted, and X, R¹ and R² are defined as above and
d) optionally removing a protecting group from a compound according to formula VI, provided that X is NR³, wherein R³ is *tert*-butyloxycarbonyl or another protecting group, to obtain a compound according to formula XIII 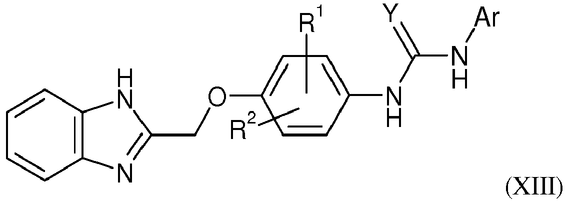 wherein Y, X, Ar, R¹ and R² are defined as above.

15. A process according to claim 14, wherein in step c) a compound according to formula VI is obtained, wherein Y is S 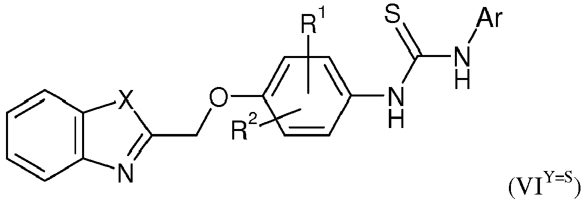
wherein R¹ and R² are independently selected out of H, halogen, C1 to C3 alkyl, halogenated C1 to C3 alkyl, C1 to C3 alkyloxy, or halogenated C1 to C3 alkyloxy;
Ar is an aryl moiety, preferably phenyl or naphthyl, or a moncyclic- or bicyclic heteroaryl moiety, wherein optionally the aryl or heteroaryl moiety is further substituted;
X is O, S, or NR³, wherein R³ is H, C1 to C3 alkyl, halogenated C1 to C3 alkyl, or *tert*-butyloxycarbonyl; and
and further comprising the steps of
e) treating the compound obtained in step c) with methyliodid to obtain a S-methylated isothiourea compound according to formula XIV 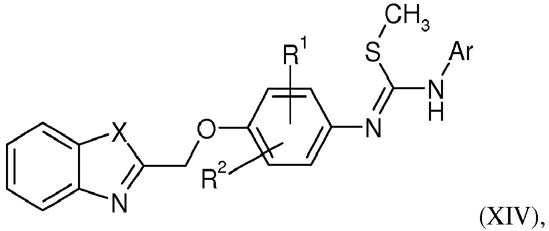
wherein X, Ar, R¹ and R² are defined as above and subsequently treating the S-methylated isothiourea compound with silver nitrate,
or treating the compound obtained in step c) with methanesulfonyl chloride in presence of a base,
wherein one of the two alternative treatments results in a carbodiimide according to formula XV 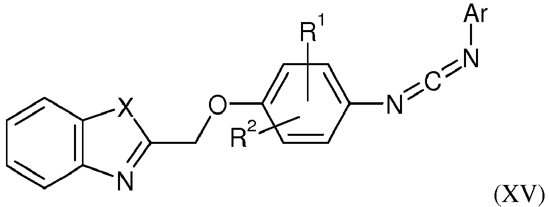 wherein X, Ar, R¹ and R² are defined as above,
f) treating the carbodiimide obtained in step e) with an amine according to formula XVI or an amine according to formula XVII 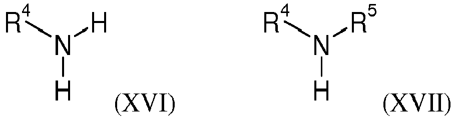 wherein R⁴ and R⁵ are independently selected out of H or C1 to C3 alkyl, to obtain a compound according to formula II 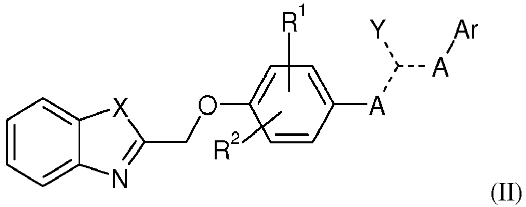
wherein X, Ar, R¹ and R² are defined as above and
wherein if the carbodiimide compound was treated with an amine according to formula XVI, then Y is NR⁴, both A are NH, and the punctuated bonds represent a double bond to Y and single bonds to each A, or
wherein if the carbodiimide compound was treated with an amine according to formula XVII, then Y is N(R⁴)R⁵, one A is N, the other A is NH, and the punctuated bonds represent a double bond to the A being N, a single bond to the A being NH and a single bond to Y;
wherein those further steps e) and f) are conducted after step c) and before the optional step d).
